# EUROPEAN PATENT APPLICATION

(11) **EP 4 552 662 A1**
(43) Date of publication of application: **14.05.2025**
(21) Application number: 24209223.7
(22) Date of filing: 28.10.2024
(51) Int. Cl.: A61L 31/02, A61L 31/08, A61L 31/14

(54) **METAL WIRE FOR MEDICAL DEVICE, MEDICAL DEVICE, AND METHOD FOR MANUFACTURING METAL WIRE FOR MEDICAL DEVICE**

(30) Priority: 09.11.2023 JP 2023191450; 25.06.2024 JP 2024101898
(71) Applicant: ASAHI INTECC CO., LTD., Seto-shi, Aichi, 489-0071 (JP)
(72) Inventor: CHIKAOKA, Sora, Seto-shi, Aichi, 4890071 (JP); USHIDA, Keisuke, Seto-shi, Aichi, 4890071 (JP); HOSHINO, Narumi, Seto-shi, Aichi, 4890071 (JP)
(74) Representative: Winter, Brandl - Partnerschaft mbB

(57) **Abstract**

This metal wire for a medical device includes: a prescribed region having a coating film exhibiting at least a prescribed color; and a specific region having a coating film exhibiting at least a specific color that is different from the prescribed color.

## Description

### INCORPORATION BY REFERENCE

The present application claims the benefit of Japanese Patent Application No. 2023-191450 filed on November 9, 2023, and the benefit of Japanese Patent Application No. 2024-101898 filed on June 25, 2024, the disclosures of which are incorporated by reference in their entirety.

### TECHNICAL FIELD

The present disclosure relates to a metal wire for a medical device, a medical device, and a method for manufacturing a metal wire for a medical device.

### BACKGROUND ART

A guide wire used when inserting a catheter or the like into a blood vessel is known. In such a guide wire, a wire (metal wire) made of a superelastic alloy, such as nickel-titanium alloy, is sometimes used as a core material. A superelastic alloy has the property of having a low susceptibility to deformation. It is known that nickel-titanium alloy loses its superelastic properties when subjected to heat treatment, making it more susceptible to deformation. For example, WO 2020/161832 and JP 2017-153615 A disclose guide wires that use such a property to improve the shaping performance by performing heat treatment with respect to a metal wire. A "metal wire" is also referred to as a core, a wire, a core wire, a core shaft, or a metal wire for a medical device.

### SUMMARY OF INVENTION

### TECHNICAL PROBLEM

In this way, a metal wire is sometimes processed by using changes in the physical properties of a metal caused by heat treatment. Examples of methods of performing heat treatment of a metal wire include annealing in an electric furnace, and heat treatment using a laser. The patent documents mentioned above give no consideration to changing the degree of susceptibility to deformation.

Such a problem is not limited to the vascular system, and is common to medical devices inserted into various organs inside the human body, such as the lymphatic system, biliary system, urinary system, respiratory system, digestive system, secretory glands, and reproductive organs, as well as to metal wires used in such medical devices.

### SOLUTION TO PROBLEM

The present disclosure has been made to solve at least part of the problem described above, and can be implemented as the following aspect.
(1) According to an aspect of the present disclosure, a metal wire for a medical device is provided. The metal wire for a medical device includes: a prescribed region having a coating film exhibiting a prescribed color; and a specific region having a coating film exhibiting a specific color.

### BRIEF DESCRIPTION OF DRAWINGS

Figs. 1A-1D are each a diagram that describes a shape test.
Fig. 2 is a table showing the physical properties of each region of a metal wire.
Fig. 3 is an explanatory diagram illustrating a configuration of a medical device according to a first embodiment.
Fig. 4 is a diagram showing the relationship between the thickness and color of a coating film.
Figs. 5A and 5B are each a diagram showing an example of a distal end portion of a metal wire, wherein Fig. 5B includes a grayscale drawing and a color drawing.
Figs. 6A and 6B are each a diagram showing an example of the surface structure of a distal end portion of a metal wire.
Figs. 7A and 7B are each an enlarged diagram of the distal end portion of a metal wire.
Fig. 8 is a flow chart showing a method for manufacturing a medical device.
Figs. 9A and 9B are each a diagram showing an example of a distal end portion of a metal wire according to a second embodiment, wherein Fig. 9B includes a grayscale drawing and a color drawing.
Figs. 10A and 10B are each a diagram showing an example of a distal end portion of a metal wire according to a third embodiment, wherein Fig. 10B includes a grayscale drawing and a color drawing.
Figs. 11A and 11B are each a diagram showing an example of a distal end portion of a metal wire according to a fourth embodiment, wherein Fig. 11B includes a grayscale drawing and a color drawing.
Figs. 12A and 12B are each a diagram showing an example of a distal end portion of a metal wire according to a fifth embodiment, wherein Fig. 12B includes a grayscale drawing and a color drawing.
Figs. 13A and 13B are each a diagram showing an example of a distal end portion of a metal wire according to a sixth embodiment, wherein Fig. 13B includes a grayscale drawing and a color drawing.
Figs. 14A and 14B are each a diagram showing an example of a distal end portion of a metal wire according to a seventh embodiment, wherein Fig. 14B includes a grayscale drawing and a color drawing.
Figs. 15A and 15B are each a diagram showing an example of a distal end portion of a metal wire according to an eighth embodiment, wherein Fig. 15B includes a grayscale drawing and a color drawing.
Figs. 16A and 16B are each an enlarged view of a distal end portion of a metal wire according to a ninth embodiment.
Fig. 17 is a flow chart showing a method for manufacturing a medical device according to the ninth embodiment.
Figs. 18A and 18B are each an enlarged view of a distal end portion of a metal wire according to a tenth embodiment.
Fig. 19 is a flow chart showing a method for manufacturing a medical device according to the tenth embodiment.
Figs. 20A and 20B are each an enlarged view of a distal end portion of a metal wire according to an eleventh embodiment.
Fig. 21 is a line diagram showing the thickness of a coating film on a distal end portion.

### DESCRIPTION OF EMBODIMENTS

The shaping performance of a metal wire can be improved by performing heat treatment. In the following, a shape test was carried out with respect to a metal wire including a first region, a second region, and a non-heat treated portion having different physical properties. As a result, it was found that the first region was most susceptible to deformation, and the second region was less susceptible to deformation than the first region.

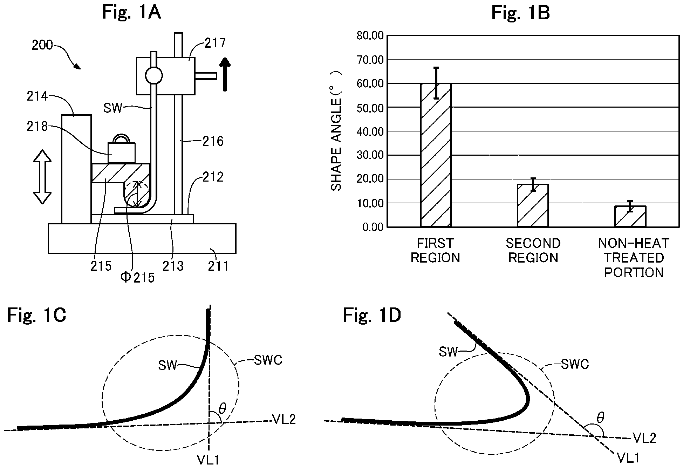

Figs. 1A-1D are each a diagram that describes the shape test. Fig. 1A shows the testing tool used in the shape test. The testing tool is a measurement device 200 that measures a shape angle Θ. The measurement device 200 includes: a base 211; a flat plate 213 that is placed on the base 211, and whose upper surface is a flat surface 212; a pin 215 that is supported by a support column 214 provided on the base 211 so as to be capable of sliding in an up-down direction; and a clamp mechanism 217 that is supported by a support rod 216 provided on the base 211 so as to be capable of sliding in the up-down direction. The pin 215 has a spherical convex portion extending downward in a vertical direction. The diameter Φ215 of the convex portion of the pin 215 is 0.5 mm. The metal wire serving as a sample SW is a wire having a first region, a second region, and a non-heat treated portion formed by changing the heat treatment conditions with respect to a wire made of nickel-titanium alloy.

The procedure of the shape test will be described. An operator sets the sample SW, being the measurement target, in the measurement device 200. Specifically, the operator fixes, to the clamp mechanism 217, a position of the sample SW which is a prescribed distance (about 250 mm to 300 mm) from the distal end, such that the sample SW extends downward from the position that has been fixed to the clamp mechanism 217 and abuts the flat surface 212, and then extends in a horizontal direction from that position on the flat surface 212. The operator presses the portion of the sample SW that is making contact with the flat surface 212 toward the flat surface 212 using the convex portion of the pin 215, and places a weight 218 on the pin 215. As a result, a load of 1 N is applied to the sample SW toward the flat surface 212 via the pin 215. In this state, the operator withdraws the sample SW in a perpendicular direction from the flat surface 212 by upwardly moving the clamp mechanism 217 along the support rod 216. The withdrawal length is about 2 mm.

For example, when the shape angle θ of the first region of the sample SW is to be measured, the operator can withdraw a center section of the first region to impart a shape to the part that has been withdrawn. At this time, the operator measures the angle of a curved section SWC of the first region of the sample SW as the shape angle θ. Figs. 1C and 1D are diagrams describing the measurement method of the shape angle θ. The operator draws a virtual extension line VL1 that extends a tangent line of a straight portion located further toward the distal end side than the curved section SWC of the sample SW, and a virtual extension line VL2 that extends a tangent line of the straight portion located further toward the proximal end side than the curved section SWC. The straight portion refers to a section of the sample SW that is not curved. The operator measures the angle θ which, of the angles formed where the virtual extension lines VL1 and VL2 intersect, is located on the far side from the curved section SWC, and takes the angle as the shape angle θ.

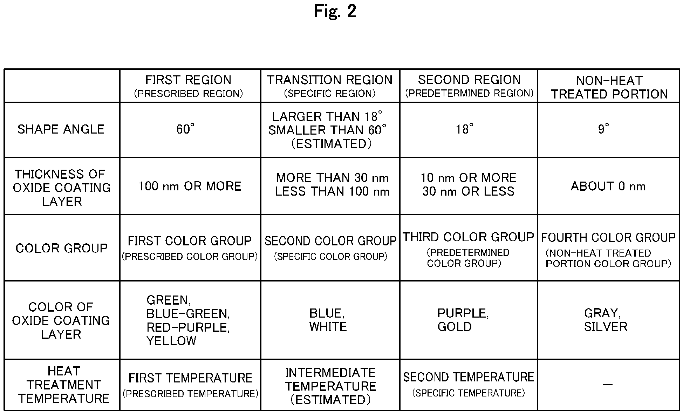

Fig. 1B shows the results of a shape test (shape angles θ of each withdrawn part of the sample SW). Fig. 2 is a table showing the physical properties of each region of a metal wire serving as the sample SW. The shape angle θ of the first region is 60°. The first region has been subjected to heat treatment at a first temperature. The thickness of an oxide coating film in the first region is 100 nm or more. The oxide coating film in the first region exhibits at least one color selected from a first color group. The first color group includes green, blue-green, red-purple, and yellow. The shape angle θ of the second region is 18°. The second region has been subjected to heat treatment at a second temperature. The thickness of the oxide coating film in the second region is 10 nm or more and 30 nm or less. The oxide coating film in the second region exhibits at least one color selected from a third color group. The third color group includes purple and gold. The shape angle θ of a transition region is estimated to be larger than 18° and smaller than 60°. The transition region is formed as a result of the heat applied to the first region being conducted along the sample SW. The heat treatment temperature of the transition region is estimated to be lower than the first temperature and higher than the second temperature. The thickness of the oxide coating film in the transition region is thicker than 30 nm and thinner than 100 nm. The oxide coating film in the transition region exhibits at least one color selected from a second color group. The second color group includes blue and white. The shape angle θ of the non-heat treated portion is 9°. The thickness of the oxide coating film in the non-heat treated portion is about 0 nm. The oxide coating film in the non-heat treated portion exhibits at least one color selected from a fourth color group. The fourth color group includes gray and silver. The magnitude relationship between the first temperature and the second temperature is as follows: first temperature > second temperature.

As is clear from Figs. 1B and 2, a correlation was obtained in which the shape angle θ (deformation angle) increases as the thickness of the oxide coating film of the metal wire increases. The thickness and color of the oxide coating film have the correlation described below. That is, it was found that the color of the oxide coating film and the susceptibility to deformation are correlated. As shown in Fig. 2, it is presumed that the shape angle θ in the transition region between the first region and the second region has the following relationship: first region > transition region > second region. The first region corresponds to a "prescribed region" in the claims. The transition region corresponds to a "specific region" in the claims. The second region corresponds to a "predetermined region" in the claims. The first temperature corresponds to a "prescribed temperature" in the claims. The second temperature corresponds to a "specific temperature" in the claims.

This has been utilized in each of the embodiments described below to successfully produce medical devices with varying degrees of susceptibility to deformation.

### <First Embodiment>

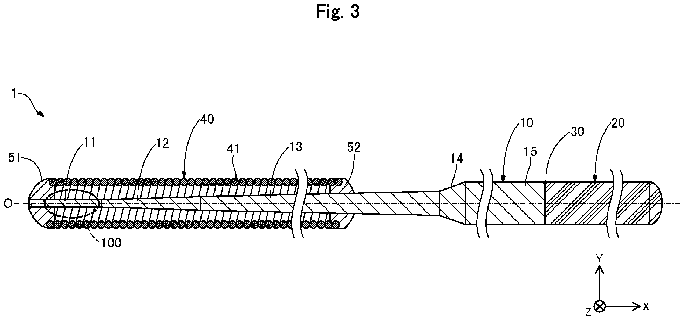

Fig. 3 is an explanatory diagram illustrating a configuration of a medical device 1 according to a first embodiment. The medical device 1 is a guide wire. The medical device 1 is used when inserting another medical device (such as a catheter) into a blood vessel or a digestive organ. The medical device 1 includes a first metal wire 10. The medical device 1 includes a second metal wire 20. The medical device 1 includes a coil 40. The medical device 1 includes a distal tip 51. The medical device 1 includes a proximal end side j oint part 52. A distal end portion 100 of the first metal wire 10 has a coating film formed by heat treatment. In the medical device 1, the susceptibility of the distal end portion 100 to deformation (shaping performance) has been improved.

For convenience of the description, Fig. 3 includes sections where the components are illustrated with relative size ratio which is different from the actual relative size ratio. Fig. 3 includes sections in which portions of the components are exaggeratedly illustrated. In Fig. 3, the axis passing through the center of the medical device is represented by an axis line O (dash-dotted line). The axis line O coincides with the axes passing through the center of each of the first metal wire 10, the second metal wire 20, and the coil 40. The axis line O may be different from the central axes of the components mentioned above. Fig. 3 shows mutually perpendicular X, Y, and Z axes. The X axis corresponds to a length direction of the medical device 1. The Y axis corresponds to a thickness direction of the medical device 1. The Z axis corresponds to a width direction of the medical device 1. The left side (negative X axis direction) of Fig. 3 is referred to as the "distal end side" of the medical device 1 and each component. The right side (positive X axis direction) of Fig. 3 is referred to as the "proximal end side" of the medical device 1 and each component. Furthermore, in the medical device 1 and each component, the end located on the distal end side is referred to as the "distal end", and the distal end and the vicinity thereof are referred to as the "distal end portion". The end located on the proximal end side is referred to as the "proximal end", and the proximal end and the vicinity thereof are referred to as the "proximal end portion". The distal end side is inserted into a living body, and the proximal end side is operated by a physician. These points are also true for the drawings following Fig. 3.

The first metal wire 10 is a metal wire for a medical device. The first metal wire 10 is arranged on the distal end side of the medical device 1. The first metal wire 10 is arranged further toward the distal end side than the second metal wire 20. For example, the first metal wire 10 is formed of nickel-titanium alloy, or an alloy of nickel-titanium and another metal. The first metal wire 10 includes, in order from the distal end side toward the proximal end side, a first portion 11, a second portion 12, a third portion 13, a fourth portion 14, and a fifth portion 15. The thickness, width, and length of each portion can be freely determined.

The first portion 11 is the portion of the first metal wire 10 that is located furthest on the distal end side. The first portion 11 is the section in which the thickness of the first metal wire 10 is the smallest. A coating film containing titanium oxide is formed on the surface of the first portion 11. The first portion 11 is subjected to press working in order to improve the susceptibility to deformation (shaping performance). The details will be described later. Hereinafter, the entire first portion 11, excluding a section on the proximal end side, will be referred to as the "distal end portion 100 of the first metal wire 10". The distal end portion 100 of the first metal wire 10 is sometimes simply referred to as the "distal end portion 100".

The second portion 12 is a section of the first metal wire 10 located between the first portion 11 and the third portion 13. The third portion 13 is a section of the first metal wire 10 located between the second portion 12 and the fourth portion 14. The fourth portion 14 is a section of the first metal wire 10 located between the third portion 13 and the fifth portion 15. The thickness of the second portion 12 becomes thinner toward the distal end. The thickness of the third portion 13 becomes thinner toward the distal end. The thickness of the fourth portion 14 becomes thinner toward the distal end. The rate of change in the thickness of the second portion 12, the rate of change in the thickness of the third portion 13, and the rate of change in the thickness of the fourth portion 14 are different from each other. The fifth portion 15 is a section of the first metal wire 10 that is located furthest on the proximal end side. The fifth portion 15 has a substantially cylindrical shape, and is the section in which the thickness of the first metal wire 10 is the largest.

In the present embodiment, "substantially constant" has the same meaning as "generally constant", and means being generally constant while allowing for deviations due to manufacturing errors and the like. Similarly, having a "substantially cylindrical shape/substantially truncated cone shape" has the same meaning as having a "generally cylindrical shape/generally truncated cone shape", and means generally having the shape while allowing for deviations due to manufacturing errors and the like. In the present embodiment, "same" and "equal" are not limited to strictly identical cases, and allow for deviations due to manufacturing errors and the like.

The second metal wire 20 is arranged on the proximal end side of the medical device 1. The second metal wire 20 is arranged further toward the proximal end side than the first metal wire 10. The second metal wire 20 is a substantially cylindrically shaped member having a constant outer diameter. The outer diameter of the second metal wire 20 is the same as the outer diameter of the fifth portion 15 of the first metal wire 10. The second metal wire 20 is formed of a material having a larger Young's modulus in a linear deformation region than the first metal wire 10, such as a stainless steel alloy such as SUS304 or SUS316.

The joint part 30 is a section where the first metal wire 10 and the second metal wire 20 have been joined by welding. The joint part 30 may be formed by a method that is different from the welding between the first metal wire 10 and the second metal wire 20, and may be formed, for example, by fixing the wires using a fixture. The member formed by joining the first metal wire 10 and the second metal wire 20 is a core shaft. The joint part 30 is formed between the proximal end surface of the fifth portion 15 of the first metal wire 10 and the distal end surface of the second metal wire 20. In the illustrated example, the joint part 30 takes the form of a flat surface that is substantially perpendicular to the axis line O. The joint part 30 may be inclined with respect to the axis line O. The first metal wire 10 and the second metal wire 20 are fixed due to the joint part 30.

The first portion 11, the second portion 12, and the distal end side of the third portion 13 of the first metal wire 10 are covered by the coil 40. The proximal end side of the third portion 13, the fourth portion 14, and the fifth portion 15 of the first metal wire 10 are not covered by the coil 40, and are exposed from the coil 40. The proximal end portion of the second metal wire 20 is used when a physician grips the medical device 1.

The coil 40 is formed by a wire 41 that has been spirally wound, and has a substantially cylindrical shape. The coil 40 may be a single-thread coil formed by winding a single wire into a single thread. The coil 40 may be a multi-thread coil formed by winding a plurality of wires into a multi-thread. The coil 40 may be a single-threaded strand coil formed by winding a strand, in which a plurality of wires have been twisted together, into a single thread. The coil 40 may be a multi-threaded strand coil formed by using a plurality of strands, in which a plurality of wires have been twisted together, and twisting the strands into a multi-thread. The wire diameter of the wire 41 of the coil 40, the outer diameter and inner diameter of the coil 40, and the length of the coil 40 can be freely determined.

The wire 41 can be formed of, for example, a stainless steel alloy such as SUS304 or SUS316, nickel-titanium alloy, piano wire, a radiopaque alloy such as nickel-chromium alloy or cobalt alloy, a radiopaque alloy of gold, platinum, tungsten, or an alloy containing these elements (such as platinum-nickel alloy), or known materials other than those mentioned above.

The distal tip 51 is provided on the distal end of the medical device 1. The distal tip 51 integrally holds together the distal end of the first portion 11 of the first metal wire 10 and the distal end of the coil 40. The proximal end side joint part 52 is oriented toward an intermediate portion of the medical device 1. The proximal end side joint part 52 integrally holds together a section of the third portion 13 of the first metal wire 10 and the proximal end of the coil 40. The distal tip 51 is formed using an arbitrary bonding agent such as a metal solder, examples of which include silver solder, gold solder, zinc, Sn-Ag alloy, and Au-Sn alloy. The proximal end side joint part 52 is formed using an arbitrary bonding agent such as a metal solder, examples of which include silver solder, gold solder, zinc, Sn-Ag alloy, and Au-Sn alloy. The distal tip 51 and the proximal end side joint part 52 may use the same bonding agent, or may use different bonding agents.

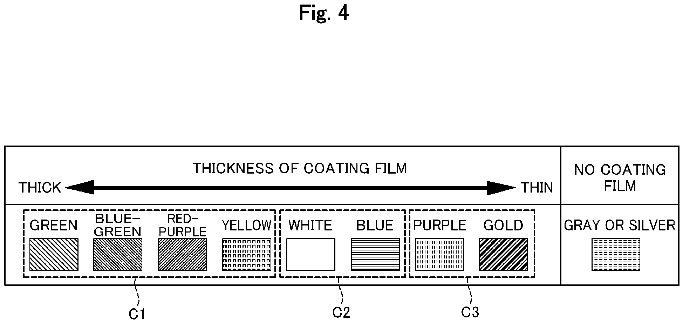

Hereinafter, the coating film formed on the distal end portion 100 of the first metal wire 10, and the detailed configuration of the distal end portion 100 will be described using Figs. 4 to 7. Like the first metal wire 10 described in Fig. 3, when heat treatment is performed with respect to a metal wire containing titanium (Ti), such as nickel-titanium alloy, or an alloy of nickel-titanium and another metal, the titanium is oxidized, which results in the formation of an oxide coating film of titanium on the surface of the metal wire. The titanium oxide coating film will also be simply referred to as a "coating film" below.

Fig. 4 is a diagram showing the relationship between the thickness and color of a coating film. The film thickness of the coating film formed on the metal wire becomes thicker as the heat treatment temperature is increased. The film thickness of the coating film formed on the metal wire becomes thinner as the heat treatment temperature is decreased. The film thickness of the coating film is related to the heat treatment temperature. The film thickness of the coating film is related to the external color of the metal wire. As shown in Fig. 4, when the coating film is "green", the thickness of the coating film is the thickest. When the coating film is "blue-green", the coating film is the second-thickest. When the coating film is "red-purple", the coating film is the third-thickest. When the coating film is "yellow", the coating film is the fourth-thickest. When the coating film is "white", the coating film is the fifth-thickest. When the coating film is "blue", the coating film is the sixth-thickest. When the coating film is "purple", the coating film is the seventh-thickest. When the coating film is "gold", the coating film is the eighth-thickest. When heat treatment is not performed and a coating film is not formed, the external color of the metal wire is either grey or silver.

In the present embodiment, "green" includes, in addition to green, other green-based colors such as lime green and sky green. "Blue-green" includes, in addition to peacock green, other colors corresponding to an intermediate color between green and blue, such as aquamarine and turquoise. "Red-purple" includes, in addition to claret, other colors corresponding to an intermediate color between red and purple, such as plum and magenta. "Yellow" includes, in addition to yellow, other yellow-based colors such as lemon yellow and topaz. "White" includes, in addition to white, other white-based colors such as silver white and oyster white. "Blue" includes, in addition to blue, other blue-based colors such as cyan and navy blue. "Purple" includes, in addition to purple, other purple-based colors such as violet and grape. "Gold" includes, in addition to antique gold, other gold-based colors such as buff and beige.

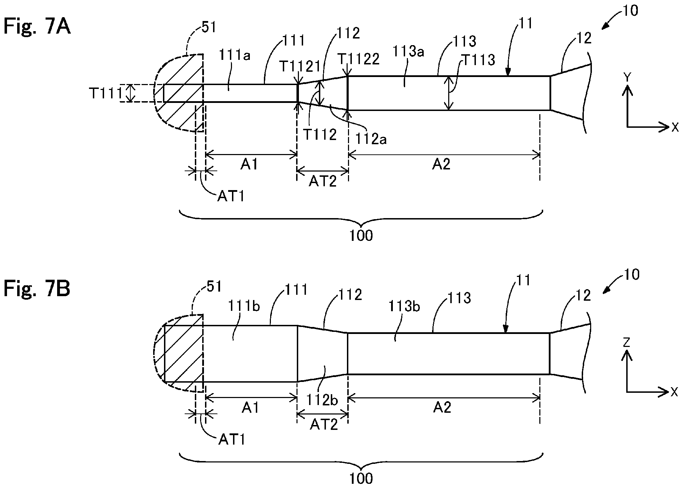

The color of the coating film can be identified by visually observing the external appearance of the heat-treated metal wire. Specifically, the operator uses a digital microscope "VHX-7000" (manufactured by Keyence Corporation) to take an external photograph of the heat-treated metal wire. Then, the operator identifies the color of the coating film by visually confirming the external photograph that has been captured. That is, the color of the coating film mentioned above is the color under the lighting environment of the digital microscope VHX-7000. The confirmation position of the oxide coating film (capture location of the external photograph) is the side surface when viewed from the Y axis direction (the side surface shown in Fig. 7B described below). The first color group C1 includes green, blue-green, red-purple, and yellow. The at least one color selected from the first color group C1 represents a prescribed color. The second color selected from the first color group represents a second prescribed color. The third color selected from the first color group represents a third prescribed color. The fourth color selected from the first color group represents a fourth prescribed color. The third color group C3 includes purple and gold. The at least one color selected from the third color group C3 represents a predetermined color. The second color selected from the third color group represents a second predetermined color. The second color group C2 includes white and blue. The two colors selected from the second color group are a specific color and a second specific color. The first color group C1 indicates a thickness of the coating film that is relatively thick. The third color group C3 indicates a thickness of the coating film that is relatively thin. The second color group C2 indicates a thickness of the coating film that is thinner than a coating film exhibiting the first color group C1, and thicker than a coating film exhibiting the third color group C3.

That is, as indicated in the upper part of Fig. 4, the thickness of the coating film in the first region is relatively thick. The thickness of the coating film in the second region is relatively thin. The thickness of the coating film in the transition region is thinner than that of the coating film in the first region and thicker than that of the coating film in the second region. In other words, the thickness of the coating film in the first region is thicker than the thickness of the coating film in the transition region. The thickness of the coating film in the first region is thicker than the thickness of the coating film in the second region. The thickness of the coating film in the transition region is thicker than the thickness of the coating film in the second region. The operator can determine the "thickness of the coating film" according to the following procedure. Specifically, the operator randomly determines three different locations inside a target region in the longitudinal direction. The operator measures the thickness of the coating film at each of the three determined locations. The operator calculates the average value of the thickness of the coating film at the three measured locations. The operator takes the calculated average value as the thickness of the coating film in the region.

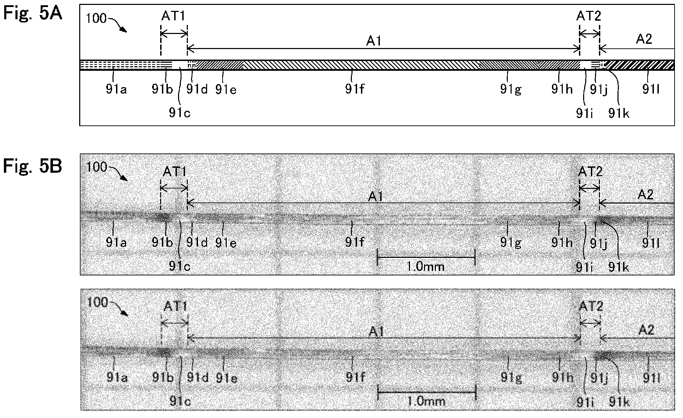

Figs. 5A and 5B are each a diagram showing an example of the distal end portion 100 of the first metal wire 10. Fig. 5A is a line diagram showing the colors of the coating film on the distal end portion 100 using hatching line types. Fig. 5B is a photograph of the distal end portion 100 including a grayscale drawing and a color drawing. The line diagram corresponds to the photograph, i.e. the black-and-white drawing (line diagram), grayscale drawing and color drawing depict the same wire and are only different graphic representations of the same wire. The line diagram and photograph in Figs. 5A and 5B each show the width direction of the flat shape of the first metal wire 10. As illustrated, the distal end portion 100 of the first metal wire 10 includes sections 91a to 911 from the distal end toward the proximal end. As the alphabet suffix advances from a, to b, to c, the position in the longitudinal direction of the first metal wire 10 moves toward the proximal end side. A non-heat treated portion on the distal end of the first metal wire 10 may be partially or completely cut and removed at the time of manufacturing the medical device 1 using the first metal wire 10. The non-heat treated portion on the distal end of the first metal wire 10 may also be used as is without being cut at the time of manufacturing the medical device 1 using the first metal wire 10. When a guide wire is produced using the first metal wire 10, the non-heat treated portion may be provided with a distal tip.

As illustrated, the section 91a is silver, which indicates that it is non-heat treated. The section 91b has a blue coating film. The section 91c has a white coating film. The section 91d has a yellow coating film. The section 91e has a red-purple coating film. The section 91f has a green coating film. The section 91g has a blue-green coating film. The section 91h has a red-purple coating film. The section 91i has a white coating film. The section 91j has a blue coating film. The section 91k has a purple coating film. The section 911 has a gold coating film. In Fig. 5A, for convenience of the illustration, the boundary between a certain section and another section that is adjacent to the certain section is clearly shown. However, as shown in Fig. 5B, the boundary between a certain section and another section may have a gradation in which the color of the coating film changes in stages. As shown in Fig. 5B, the color of the coating film in each section 91b to 91l (which includes not only the color itself but also the hue and texture), and the color gradation at the boundaries between each section 91b to 91l are exhibited due to heat treatment of the metal wire.

The "first region A1" is a region located at a center portion of the distal end portion 100 of the first metal wire 10. The first region A1 has a coating film that exhibits a color included in the first color group C1 (specifically, at least one of the colors of green, blue-green, red-purple, and yellow).

The "distal end side transition region AT1" is a region that is in a different position to the first region A1 in the longitudinal direction. The distal end side transition region AT1 is located further toward the distal end side than the first region A1 of the first metal wire 10. The distal end side transition region AT1 has a coating film that exhibits a color included in the second color group C2 (specifically, at least one of the colors of white and blue). The distal end side transition region AT1 is adjacent to the distal end of the first region A1. The "proximal end side transition region AT2" is a region that is in a different position to the first region A1 in the longitudinal direction. The proximal end side transition region AT2 is located further toward the proximal end side than the first region A1 of the first metal wire 10. The proximal end side transition region AT2 has a coating film that exhibits a color included in the second color group C2 (specifically, at least one of the colors of white and blue). The proximal end side transition region AT2 is adjacent to the proximal end of the first region A1. A region having a coating film exhibiting a color included in the second color group C2 is a transition region AT. The distal end side transition region AT1 is a transition region AT. The proximal end side transition region AT2 is a transition region AT. The coating film in the transition region AT (the distal end side transition region AT1 and the proximal end side transition region AT2) exhibits a gradation that includes a color included in the second color group C2 (white or blue). In the transition region AT, a white coating film is located on the side closer to the first region A1 than a blue coating film. The distal end side transition region corresponds to a "distal end side specific region" in the claims. The proximal end side transition region corresponds to a "proximal end side specific region" in the claims.

The "second region A2" is a region that is in a different position to the first region A1 in the longitudinal direction. The second region A2 is located on the side opposite to the first region A1 when viewed from the transition region AT (specifically, the proximal end side transition region AT2) of the first metal wire 10. The second region A2 has a coating film that exhibits a color included in the third color group C3 (specifically, at least one of the colors of purple and gold). The second region A2 is adjacent to the proximal end of the transition region AT. The second region A2 is adjacent to the proximal end of the proximal end side transition region AT2. The second region A2 is located further toward the proximal end side than the first region A1 of the first metal wire 10. The second region A2 is in a position that is separated from the first region A1 in the longitudinal direction of the first metal wire 10.

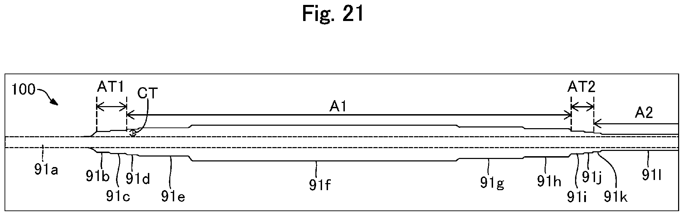

Fig. 21 is a line diagram showing the thickness of the coating film on the distal end portion 100. Fig. 21 corresponds to the line diagram of Fig. 5A. Fig. 21 shows the width direction of the flat shape of the first metal wire 10. In Fig. 21, for the purpose of the description, the first metal wire 10 is shown with a dashed line, and the coating film formed on the first metal wire 10 is illustrated with a solid line emphasizing the thickness of the coating film. As is clear from Fig. 21, the thickness CT of the coating film in the first region A1 is relatively thick. The thickness of the coating film in the second region A2 is relatively thin. The thickness of the coating film in the transition regions AT1 and AT2 is thinner than that of the coating film in the first region A1 and thicker than that of the coating film in the second region A2. In other words, the thickness CT of the coating film in the first region A1 is thicker than the thickness of the coating film in the transition regions AT1 and AT2. The thickness CT of the coating film in the first region A1 is thicker than the thickness of the coating film in the second region A2. The thickness of the coating film in the transition regions AT1 and AT2 is thicker than the thickness of the coating film in the second region A2. As illustrated, the first region A1 includes the plurality of sections 91d to 91h, which differ in the thickness of the coating film. Similarly, the transition regions AT1 and AT2 include the plurality of sections 91b, 91c, 91i and 91j, which differ in the thickness of the coating film. The second region A2 includes the plurality of sections 91k and 91l, which differ in the thickness of the coating film. The magnitude relationship of the thickness of the coating film described above, for example, also holds between the section 91d, in which the coating film is the thinnest within the first region A1, and the sections 91c and 91j, in which the coating film is the thickest within the transition regions AT1 and AT2. The same is true in comparing the transition regions AT1 and AT2 with the second region A2. The magnitude relationship of the thickness of the coating film described above, for example, also holds between the sections 91b and 91j, in which the coating film is the thinnest within the transition regions AT1 and AT2, and the section 91k, in which the coating film is the thickest within the second region A2.

As shown in Fig. 2, in the first region A1, the shape angle θ when the shape test is performed is 60°. In the transition region AT, the shape angle θ when the shape test is performed is larger than 18° and smaller than 60°. In the second region A2, the shape angle θ when the shape test is performed is 18°. In the non-heat treated portion. The shape angle θ when the shape test is performed is 9°. In the first region A1, the shape angle θ when the shape test is performed is larger than that of the transition region AT. In the first region A1, the shape angle θ when the shape test is performed is larger than that of the second region A2.

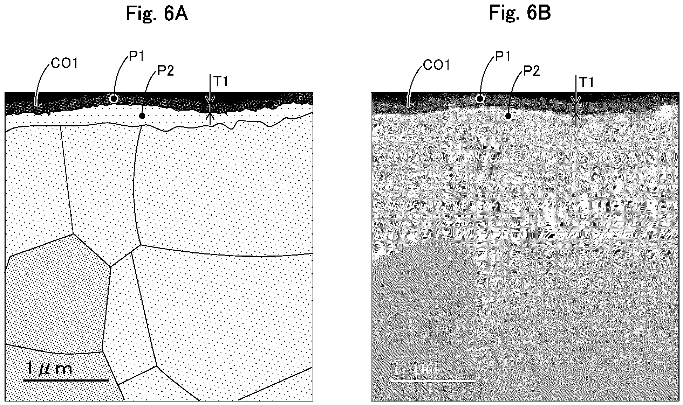

Figs. 6A and 6B are each a diagram showing an example of the surface structure of the distal end portion 100 of the first metal wire 10. Figs. 6A and 6B show an image obtained when the first region A1 of the first metal wire 10 is observed using a TEM (transmission electron microscope) "JEM-2100F" (manufactured by JEOL Ltd.). Fig. 6A is a line diagram of the first region A1 obtained by TEM. Fig. 6B is a photograph of the first region A1 obtained by TEM. Figs. 6A and 6B depict the same image.

As shown in Figs. 6A and 6B, surface segregation occurs on the surface of the heat-treated first metal wire 10 with the formation of a titanium oxide coating film, forming layers. The outermost layer is a titanium oxide coating film CO1. The thickness T1 of the coating film CO1 in the first region A1 of the first metal wire 10 is 100 nm or more. The method of obtaining the thickness T1 of the coating film CO1 will be described. The operator observes with TEM the section of the first region A1 of the first metal wire 10 exhibiting the color representing the thinnest coating film thickness (yellow in the case of the first metal wire 10 shown in Figs. 5A and 5B), and obtains the thickness T1 of the coating film CO1 by measuring the thickness of the section where the coating film is relatively thin in the image obtained by TEM.

In the first metal wire 10, the thickness T1 of the coating film CO1 in the first region A1 is thicker than the thickness of the coating film in the transition region AT. The method of obtaining the thickness of the coating film in the transition region AT will be described. The operator observes with TEM the section exhibiting the color representing the thickest coating film thickness (white in the case of the first metal wire 10 shown in Figs. 5A and 5B), and obtains the thickness of the coating film in the transition region AT by measuring the thickness at the section where the coating film is relatively thick in the image obtained by TEM.

In the first metal wire 10, the thickness of the coating film in the second region A2 is 10 nm or more and 30 nm or less. In terms of the lower limit value of the thickness of the coating film in the second region A2, the operator observes with TEM the section exhibiting the color representing the thinnest coating film thickness (gold in the case of the first metal wire 10 shown in Figs. 5A and 5B), and measures the thickness at the section where the coating film is relatively thin in the image obtained by TEM. In terms of the upper limit value of the thickness of the coating film in the second region A2, the operator observes with TEM the section exhibiting the color representing the thickest coating film thickness (purple in the case of the first metal wire 10 shown in Figs. 5A and 5B), and measures the thickness at the section where the coating film is relatively thick in the image obtained by TEM.

In the transverse cross-sectional view of the first metal wire 10 shown in Figs. 6A and 6B, an arbitrary position in the outermost layer (titanium oxide coating film CO1) is defined as a first position P1. An arbitrary position in the second layer from the outside is defined as a second position P2. At this time, the titanium content in the first position P1 is greater than the titanium content in the second position P2 (titanium: first position P1 > second position P2). The nickel (Ni) content in the second position P2 is greater than the nickel content in the first position P1 (nickel: first position P1 < second position P2). The titanium content refers to a mass content. The nickel content refers to a mass content.

The uneven distribution of titanium and nickel mentioned above is due to the surface segregation of titanium that occurs toward the surface of the first metal wire 10 as a result of heat treatment. In Figs. 6A and 6B, the titanium and nickel content in the first position P1 and the second position P2 has been described with respect to the first region A1 of the first metal wire 10. In the transition region AT and second region A2 of the first metal wire 10, like the first region A1, the titanium content has the following relationship: first position P1 > second position P2. Further, the nickel content has the following relationship: first position P1 < second position P2.

Figs. 7A and 7B are each an enlarged diagram showing the distal end portion 100 of the first metal wire 10. Fig. 7A represents a side view of the distal end portion 100 viewed from the Z axis direction. Fig. 7B represents a side view of the distal end portion 100 viewed from the Y axis direction. In Figs. 7A and 7B, illustration of the coil 40 has been omitted. The first portion 11 has a flat shape having a first pressed portion 111, a change-over portion 112, and a second pressed portion 113. The first pressed portion 111 is a section that has been subjected to press working with a first drawing ratio. The first pressed portion 111 has a relatively wide flat shape. The second pressed portion 113 is a section that has been subjected to press working with a second drawing ratio that is smaller than the first drawing ratio. The second pressed portion 113 is closer to a cylinder than the first pressed portion 111. The second pressed portion 113 has an elliptical cylindrical shape. The change-over portion 112 is a section located between the first pressed portion 111 and the second pressed portion 113 whose drawing ratio and outer shape gradually change.

The thickness T111 of the first pressed portion 111 is thinner than the thickness T113 of the second pressed portion 113. The thickness T1121 of the change-over portion 112 at the distal end of the change-over portion 112 is equal to the thickness T111 of the first pressed portion 111. The thickness T1122 of the change-over portion 112 at the proximal end of the change-over portion 112 is equal to the thickness T113 of the second pressed portion 113. The thickness T112 of the change-over portion 112 becomes gradually thicker from the distal end to the proximal end. The thickness of a pressed portion refers to the vertical distance from one main surface to the other main surface of the distal end portion 100 of the first metal wire 10, where the surface having the larger area is the main surface, and the surface perpendicular to the main surface is the side surface.

A coating film formed by heat treatment is formed on the first pressed portion 111, the change-over portion 112, and the entire second pressed portion 113 except for the proximal end portion. In the illustrated example, the distal end side of the distal end portion of the first pressed portion 111 is a non-heat treated portion. The proximal end side of the distal end portion of the first pressed portion 111 is formed having the distal end side transition region AT1. The first region A1 is formed from the center portion of the first pressed portion 111 up to the proximal end. The proximal end side transition region AT2 is formed from the distal end of the change-over portion 112 to the proximal end. The second region A2 is formed in the entire second pressed portion 113 except for the proximal end portion. The thickness of the first metal wire 10 in the first region A1 is thinner than the thickness of the first metal wire 10 in the second region A2. The thickness of the first metal wire 10 in the proximal end side transition region AT2 becomes gradually thicker from the distal end to the proximal end. The thickness of the first metal wire 10 in the first region A1 is thinner than the thickness of the first metal wire 10 in the proximal end side transition region AT2. The thickness of the first metal wire 10 in the proximal end side transition region AT2 is thinner than the thickness of the first metal wire 10 in the second region A2. The relationship between the first region A1, the transition regions AT, the second region A2, the first pressed portion 111, the change-over portion 112, and the second pressed portion 113 is merely an example, and may be freely changed.

Generally, in a guide wire, the outer diameter of the core wire is made thinner with a tapered shape toward the distal end to reduce the distal end load, and the outer diameter of the coil body is made thinner with a tapered shape toward the distal end to improve the penetration characteristics. In such a case, the bending strain is reduced due to the reduction in the thickness of the metal. Therefore, the shape angle becomes smaller towards the distal end of the guide wire. Even if the outer diameter of the core wire and the coil body is made uniform, the guide wire will simply have a uniform shape angle from the distal end to the proximal end. Therefore, a conventional guide wire often has a shaped form in which the shape angle becomes smaller toward the distal end, or a shaped form in which the shape angle is uniform. When a guide wire is produced using the first metal wire 10 of the present embodiment, unlike a conventional guide wire, the shape angle of the guide wire can be made larger toward the distal end even when the thickness of the core wire decreases toward the distal end. Therefore, in a guide wire using the first metal wire 10 of the present embodiment, the selectivity for small branched blood vessels such as peripheral blood vessels can be improved.

In the distal end portion 100 of the first metal wire 10, the coating film (the first region A1, the transition region AT, and the second region A2) is formed in the thickness direction of the flat shape of the first metal wire 10. In other words, the coating film is formed on thickness direction surfaces 111a, 112a and 113a of the first metal wire 10. In the distal end portion 100 of the first metal wire 10, the coating film (the first region A1, the transition region AT, and the second region A2) is formed in the width direction of the flat shape of the first metal wire 10. In other words, the coating film is formed on width direction surfaces 111b, 112b and 113b of the first metal wire 10.

The distal tip 51 (dashed line) is attached further toward the distal end side of the distal end portion 100 of the first metal wire 10 than the first region A1. Specifically, the distal tip 51 is attached to the distal end side transition region AT1, which is provided further toward the distal end side than the first region A1. In the illustrated example, the proximal end of the distal tip 51 is located slightly further toward the distal end side than the proximal end of the distal end side transition region AT1. The proximal end of the distal tip 51 may be in an arbitrary position inside the area of the distal end side transition region AT1.

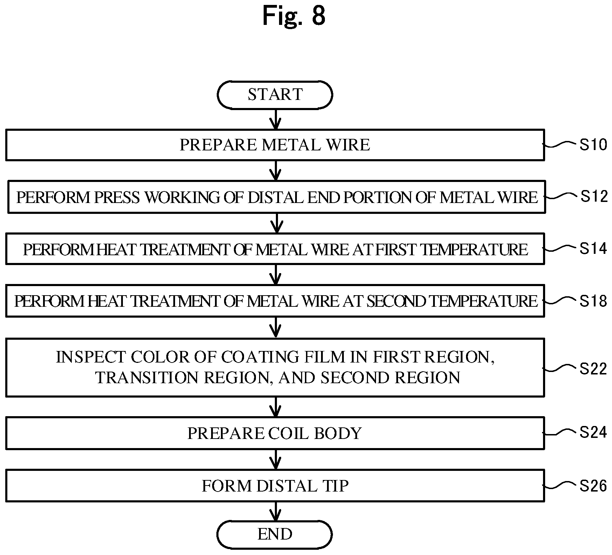

Fig. 8 is a flow chart showing a method for manufacturing the medical device 1. In step S10, the operator prepares a metal wire made of nickel-titanium alloy. The metal wire may be made of an alloy of nickel-titanium and another metal. In step S12, the operator performs press working of the distal end portion of the metal wire. In step S12, as described in Figs. 7A and 7B, press working is performed a plurality of times to form the first pressed portion 111, the change-over portion 112, and the second pressed portion 113. The press working may be performed only once in step S12.

In step S14, the operator performs heat treatment of the first region A1 at the first temperature. The heat treatment is performed by "laser heat treatment", in which a high-output laser is irradiated to heat the metal wire. The first temperature may be freely determined. The heat treatment may be performed by another heat treatment method (such as heat treatment using a heating furnace). As a result of step S14, a coating film is formed in the first region A1 and the transition region AT (the distal end side transition region AT1 and the proximal end side transition region AT2). That is, the coating film in the transition region AT (the distal end side transition region AT1 and the proximal end side transition region AT2) is formed as a result of the heat from the laser irradiated with respect to the first region A1 being conducted along the metal wire. The degree of heat treatment in the first region A1 is greater than the degree of heat treatment in the transition region AT. The degree of susceptibility of the first region A1 to deformation becomes greater than that of the transition region AT. Step S14 is a step for forming the coating film in the first region A1 and the transition region AT. The color of the coating film in the first region A1 is a color included in the first color group C1. The color of the coating film in the transition region AT is a color included in the second color group C2. The color of the coating film in the first region A1 and the color of the coating film in the transition region AT is exhibited as a result of the first heat treatment.

In step S 18, the operator performs heat treatment of the second region A2 at the second temperature. The second region A2 is located further toward the proximal end side than the first region A1. The second region A2 is located further toward the proximal end side than the proximal end side transition region AT2. The heat treatment at the second temperature is performed by laser heat treatment. The heat treatment at the second temperature may be performed by another heat treatment method (such as heat treatment using a heating furnace). The second temperature is lower than the first temperature (first temperature > second temperature). The first temperature and the second temperature can be measured by measuring the temperature of the metal wire at the time of heat treatment by using a radiation thermometer. As a result of step S18, a coating film is formed in the second region A2. By performing the heat treatment at the second temperature, which is lower than the first temperature, the degree of heat treatment of the second region A2 is lower than the degree of heat treatment of the first region A1. As a result, the degree of susceptibility of the second region A2 to deformation becomes lower than that of the first region A1. Step S18 is a step for forming the coating film in the second region A2. The color of the coating film in the second region A2 is a color included in the third color group C3. The color of the coating film in the second region A2 is exhibited as a result of the second heat treatment.

In steps S14 and S18, the above relationship (first temperature > second temperature) holds regardless of whether the heat treatments are performed with the laser output being the same (output: S14 = S18) or the heat treatments are performed with the reverse magnitude relationship (output: S14 < S18). This is because, when a laser is irradiated with respect to the XZ plane of the metal wire that has been subjected to press working shown in Fig. 7B, the irradiated area in step S14 (the area of the first pressed portion 111 and the change-over portion 112) is larger than the irradiated area in step S18 (the area of the second pressed portion 113). In the present embodiment, the irradiation diameter of the laser is larger than the width of the first pressed portion 111 in Fig. 7B.

In step S22, the operator inspects the color of the coating film in the first region A1, the transition region AT, and the second region A2. Specifically, the color of the coating film is inspected according to the following procedures a1 and a2. The procedures a1 and a2 do not need to be executed consecutively. Specifically, procedure a1 may be executed before step S14. Step S22 is a step for inspecting the color of the coating film in the first region A1, the transition region AT, and the second region A2.
(a1) The operator determines that the heat treatment of step S14 has been completed normally when the color of the coating film in the first region A1 corresponds to one of the colors included in the first color group C1 (Fig. 4), and the color of the coating film in the transition region AT corresponds to one of the colors included in the second color group C2 (Fig. 4). The operator determines that the heat treatment of step S14 has not been completed normally when the color of the coating film in the first region A1 does not correspond to any of the colors included in the first color group C 1, or the color of the coating film in the transition region AT does not correspond to any of the colors included in the second color group C2.
(a2) The operator determines that the heat treatment of step S18 has been completed normally when the color of the coating film in the second region A2 corresponds to one of the colors included in the third color group C3 (Fig. 4). The operator determines that the heat treatment of step S18 has not been completed normally when the color of the coating film in the second region A2 does not correspond to any of the colors included in the third color group C3.

As a result of steps S10 to S22 above, the first metal wire 10 described in Fig. 3 is manufactured. Steps S10 to S22 represent a method for manufacturing the metal wire 10 for a medical device. The metal wire 10 for a medical device manufactured through steps S10 to S22 has a coating film exhibiting a color included in the first color group C1, a coating film exhibiting a color included in the second color group C2, and a coating film exhibiting a color included in the third color group C3 due to heat treatment of the metal wire. In the metal wire 10 for a medical device manufactured through steps S10 to S22, normal completion of the heat treatment is confirmed by inspecting the colors of the coating film (a color included in the first color group C1, a color included in the second color group C2, and a color included in the third color group C3). According to step S22, when a plurality of first metal wires 10 are manufactured, even when a plurality of metal wires having deviations in the dimensions and the surface state are used, because the magnitude of the amount of heat applied to each metal wire (the degree of heat treatment) can be accurately and easily grasped, the first metal wire 10 can be obtained with a stable quality.

In step S24, the operator prepares the coil 40. In step S26, the operator forms the distal tip 51 by joining the distal end portion 100 of the first metal wire 10 (specifically, the distal end side transition region AT1) and the distal end of the coil 40 using an arbitrary bonding agent such as a metal solder, examples of which include silver solder, gold solder, zinc, Sn-Ag alloy, and Au-Sn alloy. Because a titanium oxide coating film has the property of having low wettability with solder, if the distal tip 51 is formed on the section in which a thick titanium oxide coating film is formed (first region A1), there is a concern that the distal tip 51 may detach from the first metal wire 10 during use. Therefore, in step S26, it is preferable that the distal tip 51 is formed further toward the distal end side than the section of the first metal wire 10 in which a thick titanium oxide coating film is formed (first region A1). The section of the first metal wire 10 in which the titanium oxide coating film is not formed (non-heat treated section) has the property of having a low susceptibility to deformation due to the superelastic properties of the first metal wire 10. The section of the first metal wire 10 in which the titanium oxide coating film is formed relatively thin (distal end side transition region AT1) has the property of having a lower susceptibility to deformation than the section in which the titanium oxide coating film is formed relatively thick (first region A1). Therefore, when the distal tip 51 is formed on the section in which the titanium oxide coating film is not formed (non-heat treated section), the distal end side transition region AT1 that is adjacent to the distal tip 51 has a low susceptibility to deformation compared to the first region A1, and there is a concern that the usability during use may decrease. Consequently, in step S26, it is preferable that the distal tip 51 is formed on the section in which the titanium oxide coating film is formed relatively thin (distal end side transition region AT1), and the distal tip 51 and the first region A1 are adjacent to each other. In step S26, the distal tip 51 may be formed after removing the coating film on the distal end side transition region AT1. Finally, the operator joins the first metal wire 10 and the second metal wire 20, and forms the joint part 30.

According to the metal wire 10 for a medical device and the medical device 1 provided with the same, the first region A1, the proximal end side transition region AT2, and the second region A2, in which the degree of heat treatment, that is, the degree of susceptibility to deformation, have been changed, are provided from the distal end side toward the proximal end side, and as mentioned above, the degree of heat treatment (degree of susceptibility to deformation) increases toward the distal end side, and has the following relationship: first region A1 > transition region AT > second region A2. Consequently, a medical device 1 can be provided in which it is possible to impart a shape with a greater curvature toward the distal end side. As a result, it becomes easier for a physician to select the desired blood vessel at a branched location of the blood vessel in a procedure using the medical device 1.

As described above, according to the metal wire 10 for a medical device of the first embodiment, the first region A1 and the transition region AT are provided in which the color of the coating film is different from each other (Figs. 5A and 5B). As a result, by changing the degree of heat treatment, that is, the degree of susceptibility to deformation, it is possible to provide a metal wire 10 for a medical device in which the physical properties gradually change. Because the first region A1 and the transition region AT each have a coating film exhibiting a different color, it is possible to identify the susceptibility of the metal wire 10 for a medical device to deformation by the external appearance (color) of the first metal wire 10.

According to the metal wire 10 for a medical device of the first embodiment, the coating film of the transition region AT has a gradation that includes two of the colors included in the second color group C2 (Figs. 5A and 5B). As a result, it is also possible to achieve a gradual change in the physical properties in the transition region AT of the first metal wire 10 by changing the degree of heat treatment, that is, the degree of susceptibility to deformation. The degree in which the physical properties gradually change in the transition region AT can be identified by the external appearance (the gradation in which the color changes from white to blue) of the first metal wire 10.

In addition, according to the metal wire 10 for a medical device of the first embodiment, the second region A2 (Figs. 5A and 5B) is provided on the side opposite to the first region A1 when viewed from the transition region AT (specifically, the proximal end side transition region AT2). As a result, by further changing the degree of heat treatment, that is, the degree of susceptibility to deformation, it is possible to provide a metal wire 10 for a medical device in which the physical properties gradually change more finely. In the second region A2, because the color of the coating film is different to that of the first region A1 and the transition region AT, it is possible to identify the susceptibility of the metal wire 10 for a medical device to deformation by the external appearance (color) of the first metal wire 10.

Further, according to the metal wire 10 for a medical device of the first embodiment, because the titanium content at the first position P1 located relatively further toward the outside than the second position P2 (in other words, near the surface of the metal wire 10 for a medical device) is high, the biocompatibility and corrosion resistance of the metal wire 10 for a medical device can be improved. Also, according to the metal wire 10 for a medical device of the first embodiment, because the nickel content at the first position P1 located relatively further toward the outside than the second position P2 (in other words, near the surface of the metal wire 10 for a medical device) is low, the biocompatibility and can be improved.

In addition, according to the metal wire 10 for a medical device of the first embodiment, because the thickness of the coating film in the first region A1 is 100 nm or more, the susceptibility of the metal wire 10 for a medical device to deformation (shaping performance) can be even further improved. Further, according to the metal wire 10 for a medical device of the first embodiment, because the thickness of the coating film in the first region A1 is thicker than the thickness of the coating film in the transition region AT, the first region A1 is more susceptible to deformation than the transition region AT. Also, according to the metal wire 10 for a medical device of the first embodiment, because the thickness of the coating film in the second region A2 is 10 nm or more and 30 nm or less, the degree of susceptibility of the second region A2 to deformation is made smaller than first region A1, and it is possible to provide a metal wire 10 for a medical device having an even finer gradual change in the physical properties.

In addition, according to the medical device 1 of the first embodiment, because the distal tip 51 is attached further toward the distal end side than the first region A1 of the metal wire 10 for a medical device, the brazing operation for forming the distal tip 51 becomes easier, and it is less likely that the metal wire 10 for a medical device will become detached from the distal tip 51. As a result, the medical device 1 can be more easily manufactured, and the safety of the medical device 1 can also be improved.

In addition, according to the medical device 1 of the first embodiment, because the distal tip 51 is attached to the distal end side transition region AT1, the brazing operation for forming the distal tip 51 becomes easier, and it is less likely that the metal wire 10 for a medical device will become detached from the distal tip 51. Also, because the distal tip 51 is attached to the distal end side transition region AT1, which is provided further toward the distal end side than the first region A1, the susceptibility of the medical device 1 to deformation (shaping performance) can be improved.

Further, according to the method for manufacturing the metal wire 10 for a medical device of the first embodiment, by performing heat treatment of the metal wire (step S14), it is possible to form the first region A1 and the transition region AT, in which the color of the coating film is different from each other. As a result, it is possible to manufacture a metal wire 10 for a medical device in which the physical properties gradually change with a single heat treatment.

In addition, according to the method for manufacturing the metal wire 10 for a medical device of the first embodiment, the second region A2 is formed by performing heat treatment of the metal wire further toward the proximal end side than the first region A1 (step S18). As a result, it is possible to manufacture a metal wire 10 for a medical device in which the physical properties more gradually change due to the two heat treatments of steps S14 and S18. Because the temperature of the heat treatment for forming the second region A2 (second temperature) is lower than the temperature of the heat treatment for forming the first region A1 and the transition region AT (first temperature), the thickness of the coating film in the first region A1 and the thickness of the coating film in the second region A2 can be easily changed.

Further, according to the method for manufacturing the metal wire 10 for a medical device of the first embodiment, because the color of the coating film is different in each of the first region A1 and the transition region AT, it is possible to easily determine whether or not the heat treatment of step S14 was completed normally (step S22) by inspecting the external appearance of the metal wire (specifically, the color of the coating film in the first region A1 and the color of the coating film of the transition region AT). As a result, the metal wire 10 for a medical device can be manufactured with a stable quality. It is possible to easily determine whether or not the heat treatment of step S18 for forming the second region A2 was completed normally (step S22) by inspecting the external appearance of the first metal wire 10 (specifically, the color of the coating film in the second region A2). As a result, the metal wire 10 for a medical device can be manufactured with a stable quality.

### <Second Embodiment>

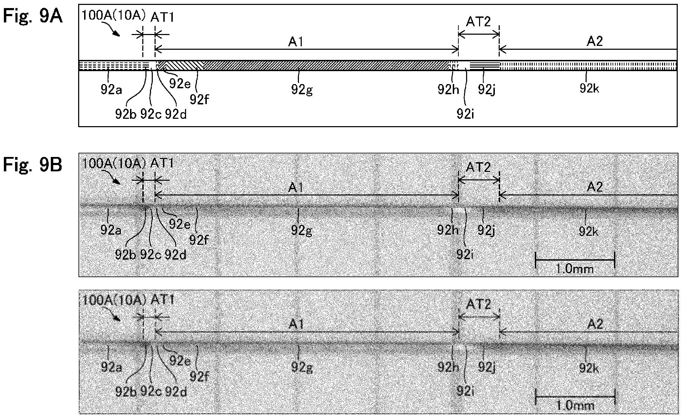

Figs. 9A and 9B are each a diagram showing an example of a distal end portion 100A of a first metal wire 10A according to a second embodiment. The medical device 1 may include the first metal wire 10A described below instead of the first metal wire 10 described in the first embodiment. The first metal wire 10A differs from the first embodiment with respect to the colors of the coating film in the first region A1, the transition region AT, and the second region A2 of the distal end portion 100A. Fig. 9A is a line diagram showing the colors of the coating film on the distal end portion 100A using hatching line types. Fig. 9B is a photograph of the distal end portion 100A including a grayscale drawing and a color drawing. The line diagram corresponds to the photograph, i.e. the black-and-white drawing (line diagram), grayscale drawing and color drawing depict the same wire and are only different graphic representations of the same wire. The line diagram and photograph in Figs. 9A and 9B each show the width direction of the flat shape of the first metal wire 10A.

As illustrated, the section 92a is silver, which indicates that it is non-heat treated. The section 92b has a blue coating film. The section 92c has a white coating film. The section 92d has a yellow coating film. The section 92e has a red-purple coating film. The section 92f has a green coating film. The section 92g has a red-purple coating film. The section 92h has a yellow coating film. The section 92i has a white coating film. The section 92j has a blue coating film. The section 92k has a purple coating film. For example, as shown in Fig. 9B, the section 92g may be a light red-purple color. The section 92k may be golden purple toward the proximal end side. For example, as shown in Fig. 9B, coating films of the same color (yellow and red-purple in the illustrated example) may repeatedly appear in the first region A1.

The center portion of the distal end portion 100A of the first metal wire 10A is the first region A1 having a coating film exhibiting colors included in the first color group C1 (specifically, green, red-purple, and yellow). The region further toward the distal end side than the first region A1 is the distal end side transition region AT1 having a coating film exhibiting colors included in the second color group C2 (specifically, white and blue). The region further toward the proximal end side than the first region A1 is the proximal end side transition region AT2 having a coating film exhibiting colors included in the second color group C2 (specifically, white and blue). The region on the side opposite to the first region A1 when viewed from the transition region AT (specifically, the proximal end side transition region AT2) is the second region A2 having a coating film exhibiting a color included in the third color group C3 (specifically, purple).

Here, various changes are possible in terms of the colors of the coating film in the first region A1, the transition region AT, and the second region A2 of the distal end portion 100A of the first metal wire 10A, the order in which the colors appear, and the combination of colors when a plurality of colors are exhibited. It is sufficient that a coating film exhibiting at least of the colors included in the first color group C1 described in Fig. 4 is formed in the first region A1. It is sufficient that a coating film exhibiting at least of the colors included in the second color group C2 described in Fig. 4 is formed in the transition region AT. It is sufficient that a coating film exhibiting at least of the colors included in the third color group C3 described in Fig. 4 is formed in the second region A2. The length of the first region A1, the transition region AT, and the second region A2 in the longitudinal direction, and the length of each section, can also be freely determined. The metal wire 10A for a medical device according to the second embodiment can also achieve the same effects as those of the first embodiment described above.

### <Third Embodiment>

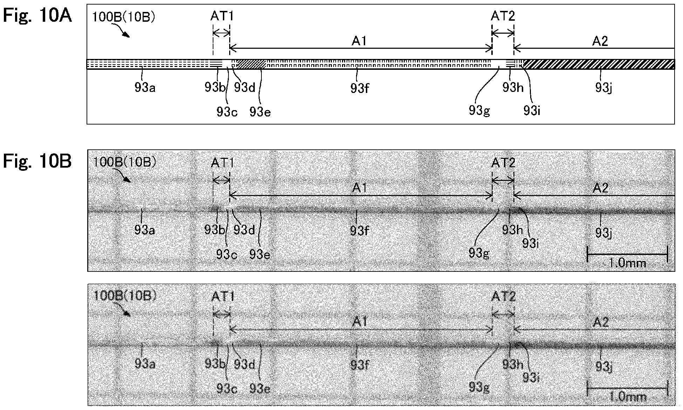

Figs. 10A and 10B are each a diagram showing an example of a distal end portion 100B of a first metal wire 10B according to a third embodiment. The medical device 1 may include the first metal wire 10B described below instead of the first metal wire 10 described in the first embodiment. The first metal wire 10B differs from the first embodiment with respect to the colors of the coating film in the first region A1, the transition region AT, and the second region A2 of the distal end portion 100B. Fig. 10A is a line diagram showing the colors of the coating film on the distal end portion 100B using hatching line types. Fig. 10B is a photograph of the distal end portion 100B including a grayscale drawing and a color drawing. The line diagram corresponds to the photograph, i.e. the black-and-white drawing (line diagram), grayscale drawing and color drawing depict the same wire and are only different graphic representations of the same wire. The line diagram and photograph in Figs. 10A and 10B each show the width direction of the flat shape of the first metal wire 10B.

As illustrated, the section 93a is silver, which indicates that it is non-heat treated. The section 93b has a blue coating film. The section 93c has a white coating film. The section 93d has a yellow coating film. The section 93e has a red-purple coating film. The section 93f has a yellow coating film. The section 93g has a white coating film. The section 93h has a blue coating film. The section 93i has a purple coating film. The section 93j has a gold coating film. For example, as shown in Fig. 10B, the section 93f may be yellow with red blemishes.

The center portion of the distal end portion 100B of the first metal wire 10B is the first region A1 having a coating film exhibiting colors included in the first color group C1 (specifically, red-purple and yellow). The region further toward the distal end side than the first region A1 is the distal end side transition region AT1 having a coating film exhibiting colors included in the second color group C2 (specifically, white and blue). The region further toward the proximal end side than the first region A1 is the proximal end side transition region AT2 having a coating film exhibiting colors included in the second color group C2 (specifically, white and blue). The region on the side opposite to the first region A1 when viewed from the transition region AT (specifically, the proximal end side transition region AT2) is the second region A2 having a coating film exhibiting a color included in the third color group C3 (specifically, purple).

Here, various changes are possible in terms of the colors of the coating film in the first region A1, the transition region AT, and the second region A2 of the distal end portion 100B of the first metal wire 10B, the order in which the colors appear, and the combination of colors when a plurality of colors are exhibited. The length of the first region A1, the transition region AT, and the second region A2 in the longitudinal direction, and the length of each section, can also be freely determined. The metal wire 10B for a medical device according to the third embodiment can also achieve the same effects as those of the first embodiment described above.

### <Fourth Embodiment>

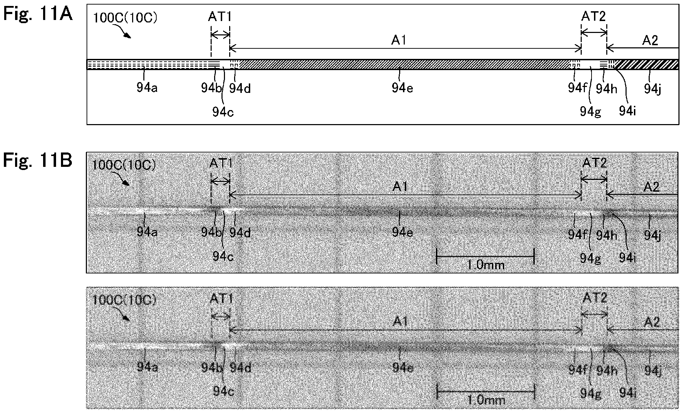

Figs. 11A and 11B are each a diagram showing an example of a distal end portion 100C of a first metal wire 10C according to a fourth embodiment. The medical device 1 may include the first metal wire 10C described below instead of the first metal wire 10 described in the first embodiment. The first metal wire 10C differs from the first embodiment with respect to the colors of the coating film in the first region A1, the transition region AT, and the second region A2 of the distal end portion 100C. Fig. 11A is a line diagram showing the colors of the coating film on the distal end portion 100C using hatching line types. Fig. 11B is a photograph of the distal end portion 100C including a grayscale drawing and a color drawing. The line diagram corresponds to the photograph i.e. the black-and-white drawing (line diagram), grayscale drawing and color drawing depict the same wire and are only different graphic representations of the same wire. The line diagram and photograph in Figs. 11A and 11B each show the width direction of the flat shape of the first metal wire 10C.

As illustrated, the section 94a is silver, which indicates that it is non-heat treated. The section 94b has a blue coating film. The section 94c has a white coating film. The section 94d has a yellow coating film. The section 94e has a red-purple coating film. The section 94f has a yellow coating film. The section 94g has a white coating film. The section 94h has a blue coating film. The section 94i has a purple coating film. The section 94j has a gold coating film. For example, as shown in Fig. 11B, the sections 94d and 94f may be a light yellow color.

The center portion of the distal end portion 100C of the first metal wire 10C is the first region A1 having a coating film exhibiting colors included in the first color group C1 (specifically, red-purple and yellow). The region further toward the distal end side than the first region A1 is the distal end side transition region AT1 having a coating film exhibiting colors included in the second color group C2 (specifically, white and blue). The region further toward the proximal end side than the first region A1 is the proximal end side transition region AT2 having a coating film exhibiting colors included in the second color group C2 (specifically, white and blue). The region on the side opposite to the first region A1 when viewed from the transition region AT (specifically, the proximal end side transition region AT2) is the second region A2 having a coating film exhibiting colors included in the third color group C3 (specifically, purple and gold).

Here, various changes are possible in terms of the colors of the coating film in the first region A1, the transition region AT, and the second region A2 of the distal end portion 100C of the first metal wire 10C, the order in which the colors appear, and the combination of colors when a plurality of colors are exhibited. The length of the first region A1, the transition region AT, and the second region A2 in the longitudinal direction, and the length of each section, can also be freely determined. The metal wire 10C for a medical device according to the fourth embodiment can also achieve the same effects as those of the first embodiment described above.

### <Fifth Embodiment>

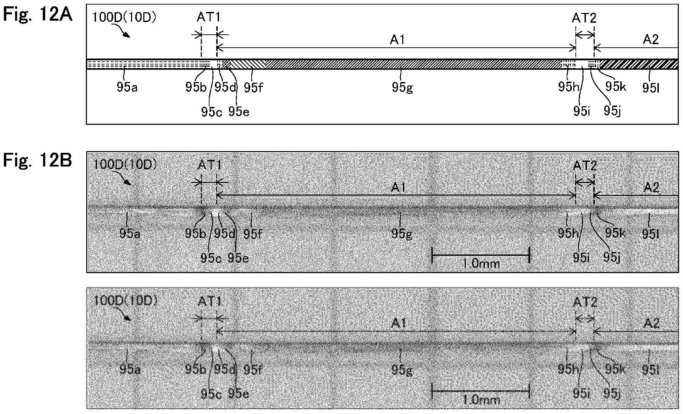

Figs. 12A and 12B are each a diagram showing an example of a distal end portion 100D of a first metal wire 10D according to a fifth embodiment. The medical device 1 may include the first metal wire 10D described below instead of the first metal wire 10 described in the first embodiment. The first metal wire 10D differs from the first embodiment with respect to the colors of the coating film in the first region A1, the transition region AT, and the second region A2 of the distal end portion 100D. Fig. 12A is a line diagram showing the colors of the coating film on the distal end portion 100D using hatching line types. Fig. 12B is a photograph of the distal end portion 100D including a grayscale drawing and a color drawing. The line diagram corresponds to the photograph i.e. the black-and-white drawing (line diagram), grayscale drawing and color drawing depict the same wire and are only different graphic representations of the same wire. The line diagram and photograph in Figs. 12A and 12B each show the width direction of the flat shape of the first metal wire 10D.

As illustrated, the section 95a is silver, which indicates that it is non-heat treated. The section 95b has a blue coating film. The section 95c has a white coating film. The section 95d has a yellow coating film. The section 95e has a red-purple coating film. The section 95f has a green coating film. The section 95g has a red-purple coating film. The section 95h has a yellow coating film. The section 95i has a white coating film. The section 95j has a blue coating film. The section 95k has a purple coating film. The section 95l has a gold coating film. For example, as shown in Fig. 12B, the sections 95d and 95i may be yellowish white. The section 95f may be pale green.

The center portion of the distal end portion 100D of the first metal wire 10D is the first region A1 having a coating film exhibiting colors included in the first color group C1 (specifically, green, red-purple, and yellow). The region further toward the distal end side than the first region A1 is the distal end side transition region AT1 having a coating film exhibiting colors included in the second color group C2 (specifically, white and blue). The region further toward the proximal end side than the first region A1 is the proximal end side transition region AT2 having a coating film exhibiting colors included in the second color group C2 (specifically, white and blue). The region on the side opposite to the first region A1 when viewed from the transition region AT (specifically, the proximal end side transition region AT2) is the second region A2 having a coating film exhibiting colors included in the third color group C3 (specifically, purple and gold).

Here, various changes are possible in terms of the colors of the coating film in the first region A1, the transition region AT, and the second region A2 of the distal end portion 100D of the first metal wire 10D, the order in which the colors appear, and the combination of colors when a plurality of colors are exhibited. The length of the first region A1, the transition region AT, and the second region A2 in the longitudinal direction, and the length of each section, can also be freely determined. The metal wire 10D for a medical device according to the fifth embodiment can also achieve the same effects as those of the first embodiment described above.

### <Sixth Embodiment>

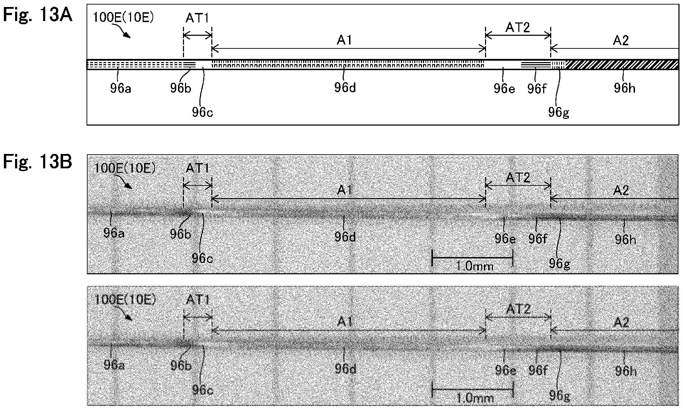

Figs. 13A and 13B are each a diagram showing an example of a distal end portion 100E of a first metal wire 10E according to a sixth embodiment. The medical device 1 may include the first metal wire 10E described below instead of the first metal wire 10 described in the first embodiment. The first metal wire 10E differs from the first embodiment with respect to the colors of the coating film in the first region A1, the transition region AT, and the second region A2 of the distal end portion 100E. Fig. 13A is a line diagram showing the colors of the coating film on the distal end portion 100E using hatching line types. Fig. 13B is a photograph of the distal end portion 100E including a grayscale drawing and a color drawing. The line diagram corresponds to the photograph, i.e. the black-and-white drawing (line diagram), grayscale drawing and color drawing depict the same wire and are only different graphic representations of the same wire. The line diagram and photograph in Figs. 13A and 13B each show the width direction of the flat shape of the first metal wire 10E.

As illustrated, the section 96a is silver, which indicates that it is non-heat treated. The section 96b has a blue coating film. The section 96c has a white coating film. The section 96d has a yellow coating film. The section 96e has a white coating film. The section 96f has a blue coating film. The section 96g has a purple coating film. The section 96h has a gold coating film. For example, as shown in Fig. 13B, the section 96d may be brownish yellow. The section 96e may be dark white.

The center portion of the distal end portion 100E of the first metal wire 10E is the first region A1 having a coating film exhibiting a color included in the first color group C1 (specifically, yellow). The region further toward the distal end side than the first region A1 is the distal end side transition region AT1 having a coating film exhibiting colors included in the second color group C2 (specifically, white and blue). The region further toward the proximal end side than the first region A1 is the proximal end side transition region AT2 having a coating film exhibiting colors included in the second color group C2 (specifically, white and blue). The region on the side opposite to the first region A1 when viewed from the transition region AT (specifically, the proximal end side transition region AT2) is the second region A2 having a coating film exhibiting colors included in the third color group C3 (specifically, purple and gold).

Here, various changes are possible in terms of the colors of the coating film in the first region A1, the transition region AT, and the second region A2 of the distal end portion 100E of the first metal wire 10E, the order in which the colors appear, and the combination of colors when a plurality of colors are exhibited. The length of the first region A1, the transition region AT, and the second region A2 in the longitudinal direction, and the length of each section, can also be freely determined. The metal wire 10E for a medical device according to the sixth embodiment can also achieve the same effects as those of the first embodiment described above.

### <Seventh Embodiment>

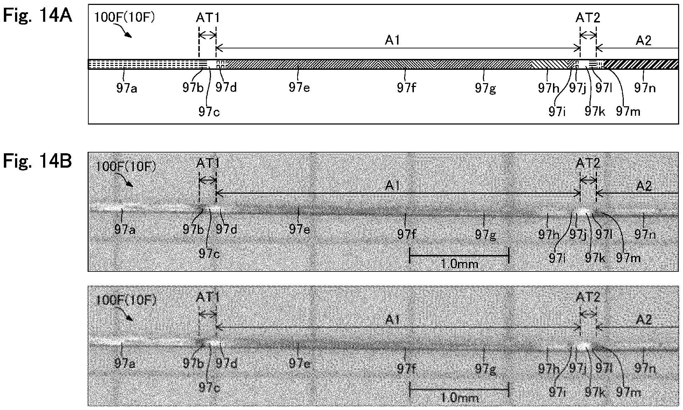

Figs. 14A and 14B are each a diagram showing an example of a distal end portion 100F of a first metal wire 10F according to a seventh embodiment. The medical device 1 may include the first metal wire 10F described below instead of the first metal wire 10 described in the first embodiment. The first metal wire 10F differs from the first embodiment with respect to the colors of the coating film in the first region A1, the transition region AT, and the second region A2 of the distal end portion 100F. Fig. 14A is a line diagram showing the colors of the coating film on the distal end portion 100F using hatching line types. Fig. 14B is a photograph of the distal end portion 100F including a grayscale drawing and a color drawing. The line diagram corresponds to the photograph, i.e. the black-and-white drawing (line diagram), grayscale drawing and color drawing depict the same wire and are only different graphic representations of the same wire. The line diagram and photograph in Figs. 14A and 14B each show the width direction of the flat shape of the first metal wire 10F.

As illustrated, the section 97a is silver, which indicates that it is non-heat treated. The section 97b has a blue coating film. The section 97c has a white coating film. The section 97d has a yellow coating film. The section 97e has a red-purple coating film. The section 97f has a blue-green coating film. The section 97g has a red-purple coating film. The section 97h has a green coating film. The section 97i has a red-purple coating film. The section 97j has a yellow coating film. The section 97k has a white coating film. The section 97l has a blue coating film. The section 97m has a purple coating film. The section 97n has a gold coating film. For example, as shown in Fig. 14B, the red-purple of the sections 97e and 97g and the blue-green of the section 97f may be mixed together to form a mottled pattern.

The center portion of the distal end portion 100F of the first metal wire 10F is the first region A1 having a coating film exhibiting colors included in the first color group C1 (specifically, green, blue-green, red-purple, and yellow). The region further toward the distal end side than the first region A1 is the distal end side transition region AT1 having a coating film exhibiting color included in the second color group C2 (specifically, white and blue). The region further toward the proximal end side than the first region A1 is the proximal end side transition region AT2 having a coating film exhibiting colors included in the second color group C2 (specifically, white and blue). The region on the side opposite to the first region A1 when viewed from the transition region AT (specifically, the proximal end side transition region AT2) is the second region A2 having a coating film exhibiting colors included in the third color group C3 (specifically, purple and gold).

Here, various changes are possible in terms of the colors of the coating film in the first region A1, the transition region AT, and the second region A2 of the distal end portion 100F of the first metal wire 10F, the order in which the colors appear, and the combination of colors when a plurality of colors are exhibited. The length of the first region A1, the transition region AT, and the second region A2 in the longitudinal direction, and the length of each section, can also be freely determined. The metal wire 10F for a medical device according to the seventh embodiment can also achieve the same effects as those of the first embodiment described above.

### <Eighth Embodiment>

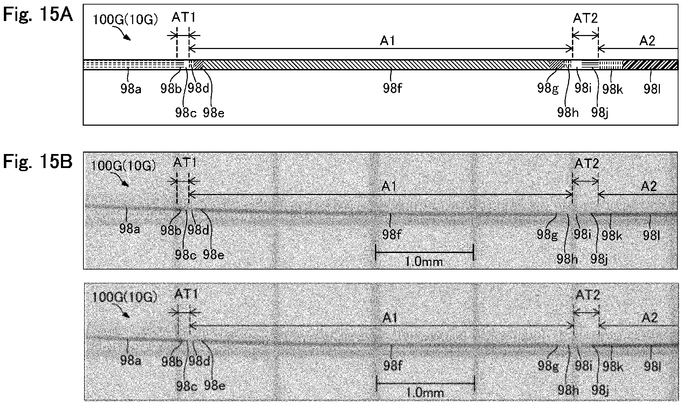

Figs. 15A and 15B are each a diagram showing an example of a distal end portion 100G of a first metal wire 10G according to an eighth embodiment. The medical device 1 may include the first metal wire 10G described below instead of the first metal wire 10 described in the first embodiment. The first metal wire 10G differs from the first embodiment with respect to the colors of the coating film in the first region A1, the transition region AT, and the second region A2 of the distal end portion 100G. Fig. 15A is a line diagram showing the colors of the coating film on the distal end portion 100G using hatching line types. Fig. 15B is a photograph of the distal end portion 100G including a grayscale drawing and a color drawing. The line diagram corresponds to the photograph, i.e. the black-and-white drawing (line diagram), grayscale drawing and color drawing depict the same wire and are only different graphic representations of the same wire. The line diagram and photograph in Figs. 15A and 15B each show the thickness direction of the flat shape of the first metal wire 10G.

As illustrated, the section 98a is silver, which indicates that it is non-heat treated. The section 98b has a blue coating film. The section 98c has a white coating film. The section 98d has a yellow coating film. The section 98e has a red-purple coating film. The section 98f has a green coating film. The section 98g has a red-purple coating film. The section 98h has a yellow coating film. The section 98i has a white coating film. The section 98j has a blue coating film. The section 98k has a purple coating film. The section 98l has a gold coating film. For example, as shown in Fig. 15B, each of the sections 98b to 98l may be a color having a relatively high brightness (bright color or pale color).

The center portion of the distal end portion 100G of the first metal wire 10G is the first region A1 having a coating film exhibiting colors included in the first color group C1 (specifically, green, red-purple, and yellow). The region further toward the distal end side than the first region A1 is the distal end side transition region AT1 having a coating film exhibiting colors included in the second color group C2 (specifically, white and blue). The region further toward the proximal end side than the first region A1 is the proximal end side transition region AT2 having a coating film exhibiting colors included in the second color group C2 (specifically, white and blue). The region on the side opposite to the first region A1 when viewed from the transition region AT (specifically, the proximal end side transition region AT2) is the second region A2 having a coating film exhibiting colors included in the third color group C3 (specifically, purple and gold).

Here, various changes are possible in terms of the colors of the coating film in the first region A1, the transition region AT, and the second region A2 of the distal end portion 100G of the first metal wire 10G, the order in which the colors appear, and the combination of colors when a plurality of colors are exhibited. The length of the first region A1, the transition region AT, and the second region A2 in the longitudinal direction, and the length of each section, can also be freely determined. The metal wire 10G for a medical device according to the eighth embodiment can also achieve the same effects as those of the first embodiment described above.

### <Ninth Embodiment>

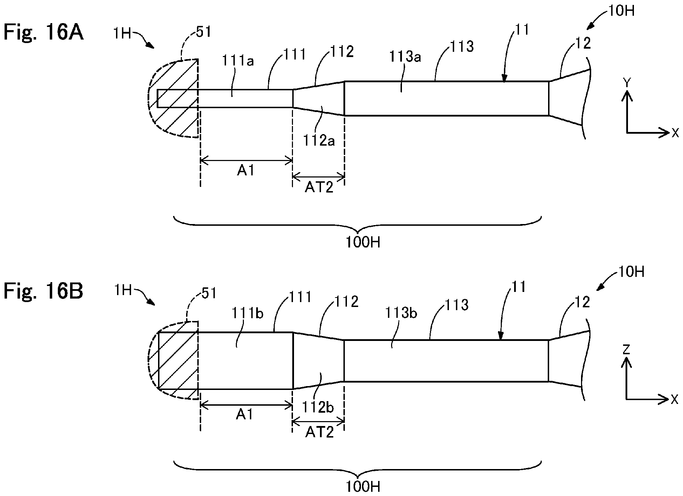

Figs. 16A and 16B are each an enlarged diagram of a distal end portion 100H of a first metal wire 10H according to a ninth embodiment. A medical device 1H according to the ninth embodiment includes the first metal wire 10H described next instead of the first metal wire 10 described in the first embodiment. The first metal wire 10H is different from the first embodiment in that the distal end side transition region AT1 and the second region A2 are not provided. Fig. 16A represents a side view of the distal end portion 100H viewed from the Z axis direction. Fig. 16B represents a side view of the distal end portion 100H viewed from the Y axis direction. In Figs. 16A and 16B, illustration of the coil 40 has been omitted.

As shown in Figs. 16A and 16B, in the medical device 1H, the distal tip 51 (dashed line) is attached further toward the distal end side of the distal end portion 100H of the first metal wire 10H than the first region A1. In the illustrated example, the proximal end of the distal tip 51 is located slightly further toward the distal end side than the distal end of the first region A1. The proximal end of the distal tip 51 may be in an arbitrary position within a range further toward the distal end side than the first region A1.

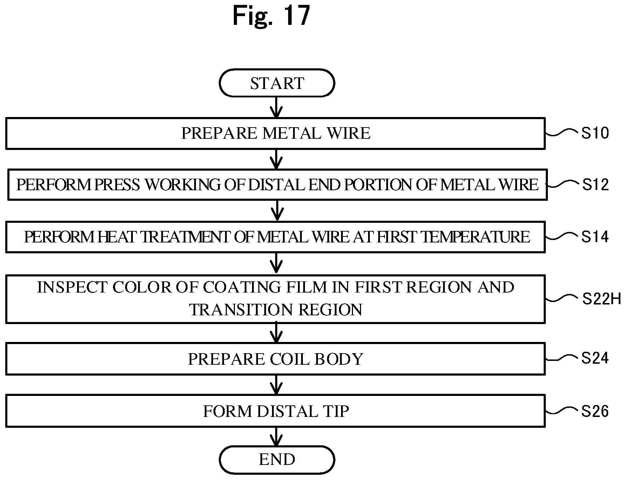

Fig. 17 is a flow chart showing a method for manufacturing the medical device 1H according to the ninth embodiment. The differences with the first embodiment shown in Fig. 8 are that step S18 is not performed, and step S22H is performed instead of step S22. In step S22H, the operator inspects the color of the coating film in the first region A1 and the transition region AT. Specifically, the operator performs only procedure a1 described in the first embodiment, and does not perform procedure a2.

In this way, various changes are possible to the configuration of the medical device 1H, and the first metal wire 10H may include the first region A1, and the transition region AT that is adjacent to the first region A1 on the proximal end side (proximal end side transition region AT2). The first metal wire 10H may include the first region A1, and the transition region AT that is adjacent to the first region A1 on the distal end side (distal end side transition region AT1). The first metal wire 10H may include the first region A1, the distal end side transition region AT1, and the proximal end side transition region AT2. The medical device 1H and the metal wire 10H for a medical device according to the ninth embodiment described above can also achieve the same effects as those of the first embodiment described above. According to the metal wire 10H for a medical device of the ninth embodiment, because the second region A2 is not included, step S18 (Fig. 8) of forming the second region A2 can be omitted, and the inspection step (Fig. 17: S22H) can be simplified. As a result, the manufacturing cost of the medical device 1H and the metal wire 10H for a medical device can be reduced.

### <Tenth Embodiment>

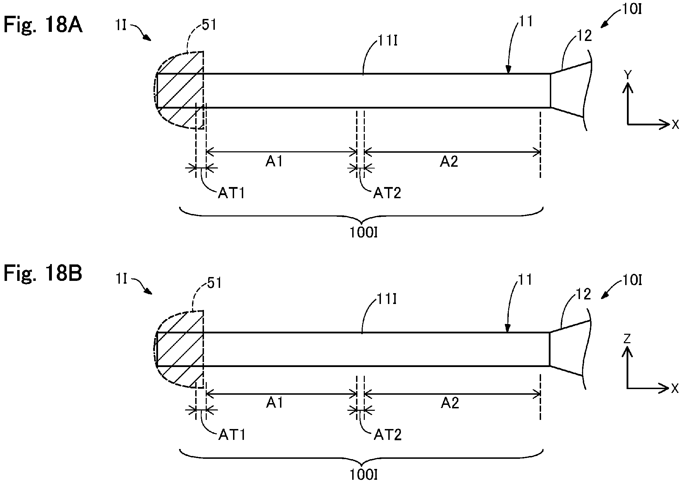

Figs. 18A and 18B are each an enlarged diagram of a distal end portion 100I of a first metal wire 10I according to a tenth embodiment. A medical device 1H according to the tenth embodiment includes the first metal wire 10I described next instead of the first metal wire 10 described in the first embodiment. The first metal wire 10I differs from the first embodiment in that the first pressed portion 111, the change-over portion 112, and the second pressed portion 113 are not provided. Fig. 18A represents a side view of the distal end portion 100I viewed from the Z axis direction. Fig. 18B represents a side view of the distal end portion 100I viewed from the Y axis direction. In Figs. 18A and 18B, illustration of the coil 40 has been omitted.

As shown in Figs. 18A and 18B, press working has not been performed with respect to the distal end portion 100I of the first metal wire 10I, and the first portion 11I has a cylindrical shape. In other words, the shape of the first portion 11I when viewed from the Z axis direction and the shape when viewed from the Y axis direction are the same.

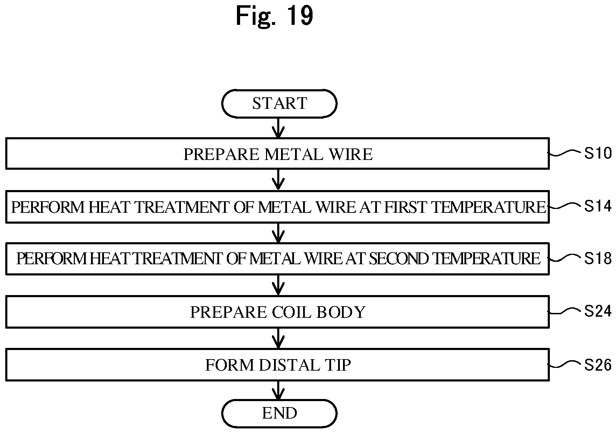

Fig. 19 is a flow chart showing a method for manufacturing the medical device 1I according to the tenth embodiment. The difference with the first embodiment shown in Fig. 8 is that steps S12 and S22 are not performed. That is, according to the present manufacturing method, heat treatment is performed without press working of the metal wire. According to the present manufacturing method, inspection of the color of the coating film in the first region A1, the transition region AT, and the second region A2 is not performed.

In this way, various changes are possible to the configuration of the medical device 1I, and the distal end portion 100I of the first metal wire 10I (first portion 11I of the first metal wire 10I) may be a round wire that has not been pressed. The medical device 1I and the metal wire 10I for a medical device according to the tenth embodiment described above can also achieve the same effects as those of the first embodiment described above. According to the metal wire 10I for a medical device of the tenth embodiment, because the press working step S12 (Fig. 8) and the inspection step S22 (Fig. 8) can be omitted, the manufacturing cost of the medical device 1I and the metal wire 10I for a medical device can be reduced.

### <Eleventh Embodiment>

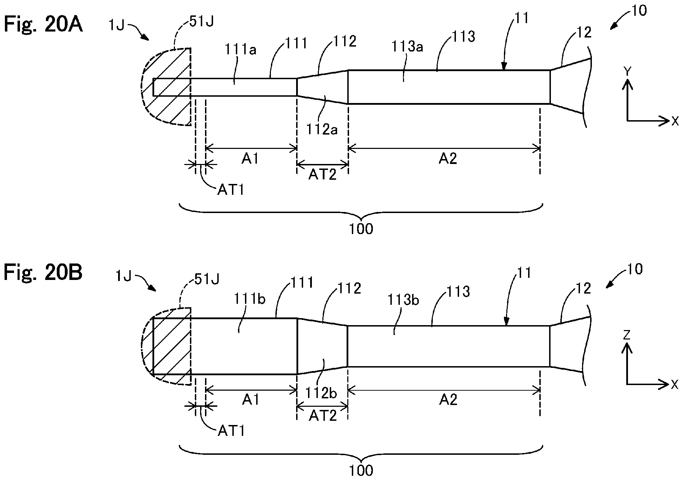

Figs. 20A and 20B are each an enlarged diagram of a distal end portion 100 of a first metal wire 10 according to an eleventh embodiment. The medical device 1J according to the eleventh embodiment includes the distal tip 51J described next instead of the distal tip 51 described in the first embodiment. The distal tip 51J differs from the first embodiment in the attachment position to the first metal wire 10. Fig. 20A represents a side view of the distal end portion 100 viewed from the Z axis direction. Fig. 20B represents a side view of the distal end portion 100 viewed from the Y axis direction. In Figs. 20A and 20B, illustration of the coil 40 has been omitted.

As shown in Figs. 20A and 20B, the distal tip 51J (dashed line) is attached further toward the distal end side of the distal end portion 100 of the first metal wire 10 than the first region A1, and further toward the distal end side than the distal end side transition region AT 1. In the illustrated example, the proximal end of the distal tip 51J is located slightly further toward the distal end side than the distal end of the distal end side transition region AT1.

In this way, various changes are possible to the configuration of the medical device 1J, and the distal tip 51J may be provided in an arbitrary position of the distal end portion 100 of the first metal wire 10. In the illustrated example, the distal tip 51J is attached further toward the distal end side than the distal end side transition region AT1. However, the distal tip 51J may be provided in the first region A1 (for example, on the distal end portion of the first region A1). The medical device 1J according to the eleventh embodiment can also achieve the same effects as those of the first embodiment described above.

### <Modifications of Present Embodiment>

The present disclosure is not limited to the embodiments described above, and may be implemented in various forms without departing from the gist of the present disclosure, and for example, the following modifications are also possible.

### [Modification 1]

In the first to eleventh embodiments described above, the configurations of the medical devices 1, 1H, 1I and 1J have been illustrated. However, various changes are possible to the configuration of the medical device 1. For example, the medical device 1 may further include a coating layer formed of either a hydrophilic resin or a hydrophobic resin on the surfaces of the first metal wire 10, the second metal wire 20, and the coil 40. For example, the length of the coil 40 in the longitudinal direction, or in other words, the range over which the coil 40 covers the first metal wire 10, may be freely changed. For example, the medical device 1 may further include an intermediate joint part between the distal tip 51 and the proximal end side joint part 52 for joining the first metal wire 10 and the coil 40. For example, various changes are possible to the shape of the second metal wire 20. The medical device 1 does not have to include the second metal wire 20. The medical device 1 does not have to include the coil 40.

### [Modification 2]

In the first to eleventh embodiments described above, the configurations of the metal wires 10, and 10A to 10I for a medical device have been illustrated. However, various changes are possible to the configuration of the first metal wire 10. For example, the shape of the first metal wire 10 described in Fig. 3 is merely an example, and various changes are possible. For example, at least one of the second portion 12, the third portion 13, and the fourth portion 14 may be omitted. The third portion 13 may have a cylindrical shape rather than a tapered shape.

In the embodiments above, the entire first portion 11 of the first metal wire 10 excluding a section on the proximal end side corresponds to the "distal end portion 100 of the first metal wire 10". Various changes are possible to the range of the distal end portion 100. For example, the entire first portion 11 of the first metal wire 10, and a section of the second portion 12 on the distal end side may correspond to the "distal end portion 100".

In the embodiments above, color patterns of the coating film in the first region A1, the transition region AT, and the second region A2 of the distal end portion 100 of the first metal wire 10 have been illustrated. However, the color pattern of the coating film in the first region A1, the transition region AT, and the second region A2 of the distal end portion 100 may be freely changed as long as a coating film exhibiting at least one of the colors included in the first color group C1 is formed in the first region A1, a coating film exhibiting at least one of the colors included in the second color group C2 is formed in the transition region AT, and a coating film exhibiting at least one of the colors included in the third color group C3 is formed in the second region A2. The distal end portion 100 of the first metal wire 10 does not have to include the non-heat treated portion.

In the embodiments above, the thickness T1 of the coating film CO1 in the first region A1 of the first metal wire 10 is 100 nm or more. The thickness T1 of the coating film CO1 in the first region A1 may be less than 100 nm. The thickness T1 of the coating film CO1 in the first region A1 is thicker than the thickness of the coating film in the transition region AT. The thickness T1 may be thinner than the thickness of the coating film in the transition region AT, and may be the same thickness as the coating film in the transition region AT.

In the embodiments above, the thickness of the coating film in the second region A2 of the first metal wire 10 is 10 nm or more and 30 nm or less. The thickness of the coating film in the second region A2 may be thinner than 10 nm. The thickness of the coating film in the second region A2 may be thicker than 30 nm.

The results of the shape test described in Fig. 2 are merely an example. The shape angle θ when the shape test is performed may also be as follows: first region A1 = transition region AT. The shape angle θ when the shape test is performed may also be as follows: first region A1 < transition region AT. The shape angle θ when the shape test is performed may also be as follows: first region A1 = second region A2. The shape angle θ when the shape test is performed may also be as follows: first region A1 < second region A2.

In the embodiments above, an arbitrary position in the outermost layer (titanium oxide coating film CO1) is the first position P1, and an arbitrary position in the second layer from the outside is the second position P2. However, the first position P1 and the second position P2 may be freely set as long as first position P1 is further toward the outside (on the surface side of the first metal wire 10) than the second position P2. In the embodiments above, the titanium content at higher in the first position P1 than at the second position P2. The titanium content may be lower at the first position P1 than at the second position P2. The titanium content may be the same at the first position P1 and the second position P2. In the embodiments above, the nickel content is lower at the first position P1 than at the second position P2. The nickel content may be higher at the first position P1 than at the second position P2. The nickel content may be the same at the first position P1 and the second position P2.

In the embodiments above, the metal wire 10 for a medical device that is incorporated in the medical device 1 has been illustrated. However, the metal wire 10 for a medical device may be manufactured independently, or in other words, without including the second metal wire 20, the coil 40, the distal tip 51, and the proximal end side joint part 52.

### [Modification 3]

The configurations of the medical device and the metal wire for a medical device according to the first to eleventh embodiments, and the configurations of the medical device and the metal wire for a medical device according to modifications 1 and 2 above may be combined as appropriate. For example, in the first metal wires 10A to 10G according to any one of the second to eighth embodiments (which have different color patterns with respect to the first region A1, the transition region AT, and the second region A2), it is possible to combine the configuration of the ninth embodiment (no second region A2 or distal end side transition region AT1), combine the configuration of the tenth embodiment (no pressing, no inspection step), and combine the configuration of the eleventh embodiment (different position of the distal tip 51). For example, in the first metal wire 10H according to the ninth embodiment (no second region A2 or distal end side transition region AT1), it is possible to combine the configuration of the tenth embodiment (no pressing, no inspection step), and combine the configuration of the eleventh embodiment (different position of the distal tip 51). For example, in the first metal wire 10I according to the tenth embodiment (no pressing, no inspection step), it is possible to combine the configuration of the eleventh embodiment (different position of the distal tip 51).

The present mode has been described based on embodiments and modifications. The embodiment of the mode described above is intended to facilitate understanding of the present mode, and does not limit the present mode. The present mode may be modified and improved without departing from the gist and the scope of the claims, and the present mode includes equivalents thereof. If the technical features are not described as essential in the present specification, the technical features may be appropriately removed.

The present disclosure includes the following embodiments:
1) A metal wire for a medical device, comprising:
   a prescribed region having a coating film exhibiting at least a prescribed color; and
   a specific region having a coating film exhibiting at least a specific color that is different from the prescribed color.
2) The metal wire for a medical device according to 1), wherein
   the prescribed region has a larger shape angle when a shape test is performed than the specific region.
3) The metal wire for a medical device according to 1) or 2), further comprising
   a predetermined region that is adjacent to the specific region and located on a side opposite to the prescribed region when viewed from the specific region, and having a coating film exhibiting at least a predetermined color.
4) A metal wire for a medical device, comprising:
   a prescribed region having a coating film exhibiting at least a prescribed color; and
   a predetermined region having a coating film exhibiting at least a predetermined color that is different from the prescribed color.
5) The metal wire for a medical device according to 4), wherein
   the prescribed region has a larger shape angle when a shape test is performed than the predetermined region.
6) The metal wire for a medical device according to 4) or 5), further comprising
   a specific region having a coating film exhibiting at least a specific color between the prescribed region and the predetermined region.
7) The metal wire for a medical device according to any one of 1) to 3), or 6), wherein
   the coating film in the specific region further exhibits a second specific color that is different from the specific color.
8) A metal wire for a medical device, comprising:
   a prescribed region having a coating film exhibiting at least a prescribed color;
   a specific region having a coating film exhibiting at least a specific color;
   a predetermined region that is adjacent to the specific region and located on a side opposite to the prescribed region when viewed from the specific region, and having a coating film exhibiting at least a predetermined color; wherein
   a thickness of the coating film in the prescribed region is thicker than a thickness of the coating film in the specific region, and
   the thickness of the coating film in the specific region is thicker than a thickness of the coating film in the predetermined region.
9) The metal wire for a medical device according to 8), wherein
   the coating film in the specific region further exhibits a second specific color that is different from the specific color.
10) The metal wire for a medical device according to any one of 3), 6), and 8), wherein
   in a cross-sectional view of at least one region among the prescribed region, the specific region, and the predetermined region, a titanium content at a first position is higher than a titanium content at a second position, being a position further toward an inner side than the first position.
11) The metal wire for a medical device according to any one of 3), 6), 8), and 10), wherein
   in a cross-sectional view of at least one region among the prescribed region, the specific region, and the predetermined region, a nickel content at a second position is higher than a nickel content at a first position, being a position further toward an outer side than the second position.
12) The metal wire for a medical device according to any one of 1) to 11), wherein
   a thickness of the coating film in the prescribed region is 100 nm or more.
13) The metal wire for a medical device according to any one of 1) to 3), or any one of 6) to 11), wherein
   a thickness of the coating film in the prescribed region is thicker than a thickness of the coating film in the specific region.
14) The metal wire for a medical device according to any one of 3) to 6), or any one of 8), 10), and 11), wherein
   a thickness of the coating film in the prescribed region is thicker than a thickness of the coating film in the predetermined region.
15) The metal wire for a medical device according to any one of 3), 6), 8), 10), and 11), wherein
   a thickness of the coating film in the specific region is thicker than a thickness of the coating film in the predetermined region.
16) The metal wire for a medical device according to any one of 3) to 6), any one of 8) to 11), or any one of 14) to 15), wherein
   a thickness of the coating film in the predetermined region is 10 nm or more and 30 nm or less.
17) The metal wire for a medical device according to any one of 3) to 6), any one of 8) to 11), or any one of 14) to 15), wherein
   a thickness of the metal wire for a medical device in the prescribed region is thinner than a thickness of the metal wire for a medical device in the predetermined region.
18) The metal wire for a medical device according to any one of 1) to 3), any one of 6) to 11), or any one of 13) to 15), wherein
   a thickness of the metal wire for a medical device in the prescribed region is thinner than a thickness of the metal wire for a medical device in the specific region.
19) The metal wire for a medical device according to any one of 3) or 6), any one of 8) to 11), or any one of 13) to 15), wherein
   a thickness of the metal wire for a medical device increases in order from the prescribed region, the specific region, and the predetermined region.
20) The metal wire for a medical device according to any one of 1) to 19), wherein
   the metal wire for a medical device has a flat shape, and
   the coating film is formed along a thickness direction of the flat shape.
21) The metal wire for a medical device according to any one of 1) to 20), wherein
   the metal wire for a medical device has a flat shape, and
   the coating film is formed along a width direction of the flat shape.
22) The metal wire for a medical device according to any one of 1) to 21), wherein
   the metal wire for a medical device is provided with a non-heat treated portion.
23) The metal wire for a medical device according to any one of 1) to 22), wherein
   the coating film is a coating film containing titanium oxide.
24) A medical device comprising:
   a metal wire for a medical device according to any one of 1) to 23); and
   a distal tip attached further toward a distal end side than the prescribed region of the metal wire for a medical device.
25) The medical device according to 24), comprising
   a metal wire for a medical device according to any one of 1) to 3), any one of 6) to 11), any one of 13) or 15), or any one of 18) to 19), wherein
   the specific region of the metal wire for a medical device includes a distal end side specific region provided further toward a distal end side than the prescribed region, and a proximal end side specific region provided further toward a proximal end side than the prescribed region, and
   the distal tip is attached to the distal end side specific region.
26) A method for manufacturing a metal wire for a medical device, the method comprising:
   forming, by performing heat treatment of a metal wire, a coating film exhibiting at least a prescribed color in a prescribed region, and a coating film exhibiting at least a specific color in a specific region.
27) The method for manufacturing a metal wire for a medical device according to 26), further comprising
   inspecting a color of the coating film in the prescribed region and a color of the coating film in the specific region in order to determine whether or not the heat treatment was completed normally.
28) The method for manufacturing a metal wire for a medical device according to 26) or 27), wherein
   a temperature of the heat treatment for forming the prescribed region and the specific region is a prescribed temperature, and
   the method further comprises
   forming, further toward a proximal end side of the metal wire than the prescribed region, a coating film exhibiting at least a predetermined color in a predetermined region by performing heat treatment at a specific temperature, being a lower temperature than the prescribed temperature.
29) The method for manufacturing a metal wire for a medical device according to 28), further comprising
   inspecting a color of the coating film in the predetermined region in order to determine whether or not the heat treatment for forming the predetermined region was completed normally.
30) A metal wire for a medical device comprising:
   a prescribed region having a coating film exhibiting at least a prescribed color; and
   a specific region having a coating film exhibiting at least a specific color; wherein
   the coating film in the prescribed region and the coating film in the specific region are formed by performing heat treatment of the metal wire for a medical device.
31) The metal wire for a medical device according to 30), wherein
   a temperature of the heat treatment for forming the prescribed region and the specific region is a prescribed temperature, and
   the metal wire for a medical device further comprises
   a predetermined region located further toward a proximal end side of the metal wire than the prescribed region, which has a coating film exhibiting at least a predetermined color that is formed by performing heat treatment at a specific temperature, being a lower temperature than the prescribed temperature.
32) A metal wire for a medical device, comprising:
   a prescribed region having a coating film exhibiting at least a prescribed color, and containing titanium oxide;
   a specific region having a coating film exhibiting at least a specific color and a second specific color, and containing titanium oxide;
   a predetermined region that is adjacent to the specific region and located on a side opposite to the prescribed region when viewed from the specific region, and which is provided with a coating film exhibiting at least a predetermined color, and containing titanium oxide; wherein
   a thickness of the coating film in the prescribed region is thicker than a thickness of the coating film in the specific region, and
   the thickness of the coating film in the specific region is thicker than a thickness of the coating film in the predetermined region.

## Claims

1. A metal wire for a medical device, comprising:
a prescribed region having a coating film exhibiting at least a prescribed color; and
a specific region having a coating film exhibiting at least a specific color that is different from the prescribed color.

2. The metal wire for a medical device according to claim 1, wherein
the prescribed region has a larger shape angle when a shape test is performed than the specific region.

3. The metal wire for a medical device according to claim 1 or 2, further comprising
a predetermined region that is adjacent to the specific region and located on a side opposite to the prescribed region when viewed from the specific region, and having a coating film exhibiting at least a predetermined color.

4. The metal wire for a medical device according to any one of claims 1 to 3, wherein
the coating film in the specific region further exhibits a second specific color that is different from the specific color.

5. The metal wire for a medical device according to claim 3, wherein
in a cross-sectional view of at least one region among the prescribed region, the specific region, and the predetermined region, a titanium content at a first position is higher than a titanium content at a second position, being a position further toward an inner side than the first position.

6. The metal wire for a medical device according to claim 3 or 5, wherein
in a cross-sectional view of at least one region among the prescribed region, the specific region, and the predetermined region, a nickel content at a second position is higher than a nickel content at a first position, being a position further toward an outer side than the second position.

7. The metal wire for a medical device according to any one of claims 1 to 6, wherein
a thickness of the coating film in the prescribed region is 100 nm or more.

8. The metal wire for a medical device according to any one of claims 1 to 3, or any one of claims 4 to 6, wherein
a thickness of the coating film in the prescribed region is thicker than a thickness of the coating film in the specific region.

9. The metal wire for a medical device according to any one of claims 3, 5 and 6, wherein
a thickness of the coating film in the prescribed region is thicker than a thickness of the coating film in the predetermined region.

10. The metal wire for a medical device according to any one of claims 3, 5, and 6, wherein
a thickness of the coating film in the specific region is thicker than a thickness of the coating film in the predetermined region.

11. The metal wire for a medical device according to any one of claims 3, 5, 6, 9 and 10, wherein
a thickness of the coating film in the predetermined region is 10 nm or more and 30 nm or less.

12. The metal wire for a medical device according to any one of claims 3, 5, 6, 9, and 10, wherein
a thickness of the metal wire for a medical device in the prescribed region is thinner than a thickness of the metal wire for a medical device in the predetermined region.

13. The metal wire for a medical device according to any one of claims 1 to 3, any one of claims 4 to 6, or any one of claims 8 to 10, wherein
a thickness of the metal wire for a medical device in the prescribed region is thinner than a thickness of the metal wire for a medical device in the specific region.

14. The metal wire for a medical device according to any one of claims 3, 5 or 6, or any one of claims 8 to 10, wherein
a thickness of the metal wire for a medical device increases in order from the prescribed region, the specific region, and the predetermined region.

15. The metal wire for a medical device according to any one of claims 1 to 14, wherein
the metal wire for a medical device has a flat shape, and
the coating film is formed along a thickness direction of the flat shape.

16. The metal wire for a medical device according to any one of claims 1 to 15, wherein
the metal wire for a medical device has a flat shape, and
the coating film is formed along a width direction of the flat shape.

17. The metal wire for a medical device according to any one of claims 1 to 16, wherein
the metal wire for a medical device is provided with a non-heat treated portion.

18. The metal wire for a medical device according to any one of claims 1 to 17, wherein
the coating film is a coating film containing titanium oxide.

19. A medical device comprising:
a metal wire for a medical device according to any one of claims 1 to 18; and
a distal tip attached further toward a distal end side than the prescribed region of the metal wire for a medical device.

20. The medical device according to claim 19, comprising
a metal wire for a medical device according to any one of claims 1 to 3, any one of claims 4 to 6, any one of claims 8 or 10, or any one of claims 13 to 14, wherein
the specific region of the metal wire for a medical device includes a distal end side specific region provided further toward a distal end side than the prescribed region, and a proximal end side specific region provided further toward a proximal end side than the prescribed region, and
the distal tip is attached to the distal end side specific region.

21. A method for manufacturing a metal wire for a medical device, the method comprising:
forming, by performing heat treatment of a metal wire, a coating film exhibiting at least a prescribed color in a prescribed region, and a coating film exhibiting at least a specific color in a specific region.

22. The method for manufacturing a metal wire for a medical device according to claim 21, further comprising
inspecting a color of the coating film in the prescribed region and a color of the coating film in the specific region in order to determine whether or not the heat treatment was completed normally.

23. The method for manufacturing a metal wire for a medical device according to claim 21 or 22, wherein
a temperature of the heat treatment for forming the prescribed region and the specific region is a prescribed temperature, and
the method further comprises
forming, further toward a proximal end side of the metal wire than the prescribed region, a coating film exhibiting at least a predetermined color in a predetermined region by performing heat treatment at a specific temperature, being a lower temperature than the prescribed temperature.

24. The method for manufacturing a metal wire for a medical device according to claim 23, further comprising
inspecting a color of the coating film in the predetermined region in order to determine whether or not the heat treatment for forming the predetermined region was completed normally.

25. A metal wire for a medical device comprising:
a prescribed region having a coating film exhibiting at least a prescribed color; and
a specific region having a coating film exhibiting at least a specific color; wherein
the coating film in the prescribed region and the coating film in the specific region are formed by performing heat treatment of the metal wire for a medical device.

26. The metal wire for a medical device according to claim 25, wherein
a temperature of the heat treatment for forming the prescribed region and the specific region is a prescribed temperature, and
the metal wire for a medical device further comprises
a predetermined region located further toward a proximal end side of the metal wire than the prescribed region, which has a coating film exhibiting at least a predetermined color that is formed by performing heat treatment at a specific temperature, being a lower temperature than the prescribed temperature.
